# EUROPEAN PATENT SPECIFICATION

(11) **EP 0 543 482 B1**
(45) Date of publication and mention of the grant of the patent: **06.02.2002**
(21) Application number: 92307091.6
(22) Date of filing: 03.08.1992
(51) Int. Cl.: A61K 49/00

(54) **Aminocarboxylate ligands having substituted aromatic amide moieties**
Aminokarboxylat-Liganden mit substituierten, aromatischen Aminogruppen
Ligands d'aminocarboxylate ayant des groupes amides aromatiques substitués

(30) Priority: 01.08.1991 US 738998
(43) Date of publication of application: 26.05.1993
(73) Proprietor: BRACCO INTERNATIONAL B.V., 1083 HJ Amsterdam (NL)
(72) Inventor: Pillai, Radhakrishna, Kendall Park, New Jersey (US); Marinelli, Edmund R., Lawrenceville, New Jersey (US); Tweedle, Michael F., Princeton, New Jersey (US); Ranganathan, Ramachandran S., Princeton, New Jersey (US); Kang, Sang-Ihn, Cranbury, New Jersey (US)
(74) Representative: Macchetta, Francesco

(56) References cited:
- EP-A- 0 485 045
- J. CHEM. SOC., PERKIN TRANS. 2, NO. 1, PAGES 87-99, 1991, GB BROAN C ET AL. 'Structure and solution stability of indium and gallium complexes of 1,4,7-triazacyclononanetriacetate and of yttrium complexes of 1,4,7,10-tetraazac yclododecanetetraacetate and related ligands: kinetically stable complexes for use in imaging and radioimmunotherapy. X-Ray....'
- INORGANIC CHEMISTRY, VOL. 30, NO. 6, PAGE(S) 1265-1269, 1991, US D D DISCHINO ET AL. 'SYNTHESIS OF NONIONIC GADOLINIUM CHELATES USEFUL AS CONTRAST AGENTS FOR MAGNETIC RESONANCE IMAGING.'

## Description

### Background of the Invention

Metal-chelating ligands are useful in diagnostic medicine as contrast agents. X-ray imaging, radionuclide imaging, ultrasound imaging and magnetic resonance imaging can each be enhanced by the use of a metal atom bound to a chelating ligand. For example, a chelating ligand can become a radiopharmaceutical when it is prepared as a chelate complex with ^{99m}Tc, ¹¹¹In, ⁶⁷Ga, ¹⁴⁰La, ¹⁶⁹Yb, ⁶⁸Ga, ⁹⁰Y, ¹⁸⁸Re, ¹⁵³Sm or other radioactive metal ions. When a chelating ligand is complexed with the stable isotopes of the lanthanides, tantalum, bismuth or other elements with molecular weight higher than iodine, the resulting complex absorbs x-rays sufficiently to act as an x-ray contrast agent. In some cases, the agents that are useful in x-ray imaging absorb, reflect or scatter ultrasound radiation sufficiently to be used as an ultrasound agent. If a chelating ligand is complexed with a paramagnetic metal atom that has a symmetric electronic ground state (e.g., Gd⁺³, and octahedral Mn⁺², Fe⁺³, Cr⁺³) the resulting complex will be useful as a spin relaxation catalyst that is used in magnetic resonance imaging (also known as NMR imaging) as a contrast agent. If a chelating agent is complexed with a paramagnetic metal atom that has an unsymmetrical electronic ground state (e.g., dysprosium(III), holmium(III) and erbium(III), the resulting complex will be useful as a chemical shift agent in magnetic resonance imaging or in magnetic resonance in vivo spectroscopy. In addition, any paramagnetic metal ion complex may be used as a contrast agent by virtue of its magnetic susceptibility as disclosed by Villringer et al. (Magnetic Resonance in Medicine, 6, 164-174, 1988).

The chelating ligands can also be bifunctional. That is, they can bind tightly to the metal ion forming a chelate while at the same time bearing a second functionality which confers upon it desirable chemical, physical and/or biological properties. Desirable physical properties of the chelator differ depending on the diagnostic or therapeutic purpose of the metal chelate. Desirable physical properties common to all uses are high affinity for the metal ion bound to the chelator and ease of synthesis. When it is desired to use the metal chelate as a contrast media for NMR imaging or general purpose x-ray imaging, the desirable physical properties are high water solubility, and viscosity and osmolality of a formulated drug solution as close as possible to those of human blood. Further, in the specific instance of a spin relaxation catalyst, the greatest possible relaxivity is desired. Relaxivity as used herein is understood to be as the effectiveness, per mole of complex, of altering the relaxation times of the nucleii being imaged.

Human blood has an osmolality of 0.3 Osmol/kg-water. Hyperosmolality is a well known contributor to adverse patient reactions to injected contrast media, and the lower osmolality of newer x-ray agents is due to their being nonionic molecules (possessing a net zero overall charge) (Shehadi, W. H.; "Contrast media adverse reactions: occurrence, reoccurrence and distribution patterns", Radiol, 1982, 143, 11-17. Bettman, M. A.; "Angiographic contrast agents; conventional and new media compared", Am. J.Roentgen, 1982, 139, 787-794. Bettman, M. A. and Morris, T. W.; Recent advances in contrast agents, Radiol. Clin. North Am., 1986, 24, 347-357.). Many gadolinium-based NMR agents in the prior art that are useful have a net negative overall charge, and therefore their aqueous formulated solutions have high osmolality. For example, Gd(DTPA)²-where DTPA stands for diethylenetriaminepentaacetic acid is formulated for use at 0.5M in water as the N-methylglucamine salt. The osmolality of the solution is 1.6 to 2.0 Osmol/kg-water. New nonionic Gd complexes are described in U. S. 4,859,451 and 4,687,659. The preferred new gadolinium complexes of the present invention are nonionic - they are not salts. When these nonionic gadolinium complexes are formulated at 0.5M in water the osmolality of the solutions is 0.3-0.6 Osmol/kg-water. The complex should be generally inert to interaction with the body other than general tissue distribution and excretion, usually by the renal route, without, or minimally, depositing Gd metal in tissues for long periods of time. Gd complexes of macrocyclic aminocarboxylates are generally more chemically inert than Gd complexes of linear aminocarboxylates (P. Wedeking and M. Tweedle, Nucl. Med. Biol., 15, 395-402, 1988; M. Tweedle et al., Magn. Reson. Imog., 9, 409-415, 1991; and M. Tweedle, "Contrast and Contrast Agents in Magnetic Resonance Imaging", edited by P. A. Rink, European Workshop on Magnetic Resonance in Medicine, 1989) The preferred aminocarboxylate ligands for Gd are therefore members of the macrocyclic aminocarboxylate class, and are, in addition, nonionic. These properties are important to NMR imaging, but, in addition, the effectiveness of an agent for NMR imaging can be increased by altering the chemical structure so as to increase the ability of the metal chelate to affect the relaxation times of water protons.

In radiopharmaceutical imaging the doses administered are relatively small so that matching the drug formulation's physical properties to those of human blood is relatively unimportant. In this use biological specificity is more important. In particular, one could use ^{99m}Tc as the metal and a chelating ligand which is functionalized with a biologically active entity such as a bile acid, fatty acid, amino acid, peptide, protein or one of numerous chemical entities known to bind receptors in vivo. NMR contrast media may also make use of biological specificity.

In radiopharmaceutical therapy, the metal ions may be chosen from among those known in the art; for example, ⁹⁰Y ¹⁸⁸Re, ¹⁵³Sm. For this purpose the chelating ligand is generally covalently bound to a disease specific entity such as monoclonal antibody. When the metal-chelator-antibody conjugate is injected into humans, it concentrates at the disease site, usually a malignant tumor. In this use the chelating ligand must contain a reactive functionality which allows for a covalent bond to be formed between the chelating ligand and the antibody. Important characteristics of the reactive functionality are as follows: (1) it must be covalently attached to the chelator such that it does not significantly diminish the affinity of the chelator for the metal ion; (2) it must allow simple synthesis in high yield of metal-chelator-antibody conjugates, the conjugate so-formed should have maximal affinity for its antigen, such affinity being minimally diminished as a result of covalently attaching the metal-chelator; (3) it should ideally allow for rapid excretion and/or optimal dosimetry of the radioactive metal chelator in the event that the metal-chelator-antibody conjugate is decomposed or metabolized in vivo.

When the metal is non-radioactive and paramagnetic such as gadolinium (III), the bifunctional chelate is useful in magnetic resonance imaging as a contrast agent, either as a discrete molecule or bound to substances such as lipids, sugars, alcohols, bile acids, fatty acids, receptor-binding ligands, amino acids, peptides, polypeptides, proteins, and monoclonal antibodies. When the metal is radioactive, such as yttrium(III) as ⁹⁰Y, the bifunctional chelate is useful in labeling monoclonal antibodies for use in radiotherapy. When the metal is ^{99m}Tc, ¹¹¹In, ²⁰¹Tl, ⁶⁷Ga, ⁶⁸Ga or the like, the chelate is useful in radiopharmaceutical imaging.

Two general methods have been employed for making bifunctional chelates from chelating agents. In the first method one or more carboxylic acid groups of a polyamino, polycarboxylic acid chelator are activated by conversion to such activating groups as internal or mixed anhydrides, activated esters (e.g., p-nitro phenyl, N-hydroxysuccinimide, etc.) or with other derivatives known to those skilled in the art. The activated acid group is then reacted with the protein. The metal ion is then added to the protein-chelator complex.

There are two problems with this method. First, using a potential donor group, the carboxylic acid, to react with the protein can diminish the strength of the chelate and contribute to the chemical lability of the metal ion. The second problem arises because the chelating ligands have several carboxylates that are not uniquely reactive. When the chelating ligand is combined with an activating agent more than one species can result because the number and chemical position of the groups activated cannot be adequately controlled. When a mixture of such variously activated chelating ligands is added to protein, protein-chelator complexes of variable and uncertain chelating strength can be formed. Also, multiple activation of carboxylic acids on a chelator leads to intra- and inter-molecular crosslinking which is a major source of decreased immunospecificity. This problem could be overcome by separating all of the products formed from the reaction of the activating agent with the chelating ligand, but that process is very laborious and makes the overall synthesis highly inefficient.

The second method for making a bifunctional chelate is to prepare a chelating ligand with a unique reactive function, such as an isothiocyanate, attached to the chelating ligand at a position that does not substantially diminish the strength with which the chelating ligand binds the metal ion. An article entitled "Synthesis of 1-(p-isothiocyanato-benzyl) derivatives of DTPA and EDTA, Antibody Labeling and Tumor-Imaging Studies" by Martin W. Brechbiel, Otto A. Gansow, Robert W. Atcher, Jeffrey Schlom, Jose Esteban, Diane E. Simpson, David Colcher, Inorganic Chemistry, 1986, 25, 2772 is illustrative of the above second method. Also, U. S. Patent 4,885,363 describes these methods as they apply specifically to nonionic macrocyclic aminocarboxylates.

Wedeking et al., "Biodistribution and Excretion of New Gd-Complexes in Mice", Abstracts of the 8th Annual Meeting of the Society of Magnetic Resonance in Medicine, 801, 1989, have disclosed the compound 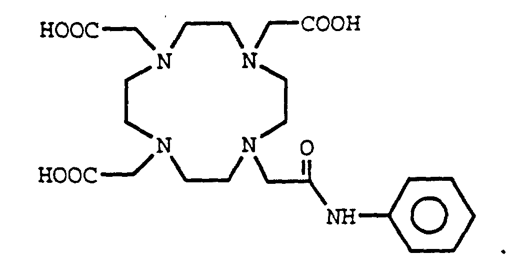 When used to chelate a paramagnetic ion, e.g., Gd, in magnetic resonance imaging, this compound was found to have poor water solubility, although acceptable relaxivity.

The chelate with ⁹⁰Y of the following structure 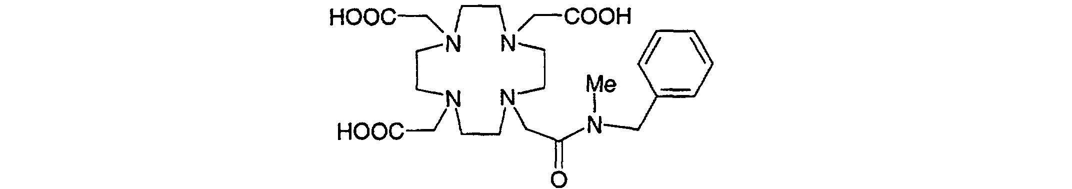 was described by Broan et al. in J. Chem. Soc. Perkin Trans. 2, 87-99, 1991, with the aim to search structures for more stable complexes for effective tumour targeting with radiolabelled monoclonal antibodies.

In EP-A-485045 tetraazacyclododecane compounds of the following general formula 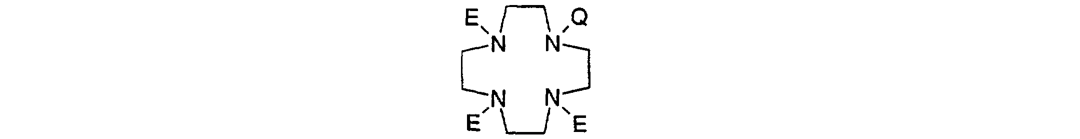 In which E and Q are defined herein and their salts with inorganic and/or organic bases, amino acids, or amino acids amides are valuable pharmaceutical agents, particularly in diagnosis applications.

### Brief Description of the Invention

This invention provides new metal-chelating ligands, and complexes thereof with metal ions.

Various aspects of the invention are set forth in the independent claims, the invention extending to the subject matter common to these claims. Preferred features of the invention are set forth in the dependent claims. Features of the preferred forms of the invention include:-
(a) metal chelate complexes that are nonionic;
(b) metal chelating ligands which when complexed with a metal heavier than iodine (e.g., Ba, Ta, Pb, Bi, Lanthanides) are effective as x-ray contrast agents;
(c) metal chelating ligands which when complexed with gamma emitting radioactive nuclide (e.g., ^{99m}Tc or ¹¹¹In) are effective as imaging radiopharmaceuticals;
(d) metal chelating ligands which when complexed with beta or alpha emitting radioactive nuclide (e.g., ⁹⁰Y, ¹⁵³Sm, ¹⁸⁸Re, ²¹²Bi) are effective as therapeutic radiopharmaceuticals ;
(e) metal-chelating ligands whose metal chelate complexes in aqueous solution have low osmolality ;
(f) metal-chelating ligands whose metal chelate complexes have low acute toxicity;
(g) metal-chelating ligands which, when complexed with a paramagnetic metal atom, are effective as relaxation catalysts in magnetic resonance imaging;
(h) bifunctional metal-chelating ligands that have the ability to covalently bind to proteins or other biologically active molecules thereby imparting biological specificity to the metal chelate complex (the conversion of the novel molecules described herein to bifunctional chelates is accomplished using the methods described above);
(i) metal complexes with increased relaxivity; and
(j) bifunctional metal-chelating ligands that are thermodynamically stable, kinetically inert and, when desired, electrically neutral.

### Detailed Description of Preferred Embodiments

The terms "alkyl" and "alkoxy" as used throughout the specification, refer to both straight and branched chain groups. Those groups having 1 to 5 carbon atoms are preferred and methyl is the most preferred alkyl group.

The term "aryl" as used throughout the specification refers to phenyl and substituted phenyl. Preferred substituted phenyl groups are those substituted with 1, 2 or 3 halogen, hydroxyl, hydroxyalkyl, alkyl, alkoxy, carbamoyl, carboxamide, acylamino or carboxyl groups.

Hydroxyalkyl refers to straight and branched alkyl bearing radicals R-OH groups such as -CH₂CH₂OH, -CH₂CH₂OHCH₂OH, CH(CH₂OH)₂ and the like. Such chemistry is well known to those skilled in the art (Sovak, M., editor Radiocontrast Agents, Springer-Venlag, 1984, pp. 1-125).

As described above, aminocarboxylate nuclei known in the art can be provided with a substituted aromatic amide moiety of formula I to provide the novel compounds of the present invention.

Exemplary novel aminocarboxylates having a substituted aromatic amide moiety include compounds of the formula

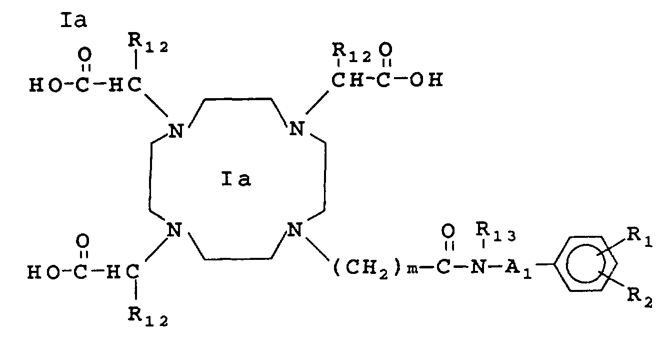

The compounds of formula Ia and salts thereof, can be complexed with a paramagnetic metal atom and used as relaxation enhancement agents for magnetic resonance imaging. These agents, when administered to a mammalian host (e.g., humans) distribute in various concentrations to different tissues, and catalyze relaxation of protons (in the tissues) that have been excited by the absorption of radiofrequency energy from a magnetic resonance imager. This acceleration of the rate of relaxation of the excited protons provides for an image of different contrast when the host is scanned with a magnetic resonance imager. The magnetic resonance imager is used to record images at various times generally before and after administration of the agents, and the differences in the images created by the agents' presence in tissues are used in diagnosis. In proton magnetic resonance imaging, paramagnetic metal atoms such as gadolinium(III), and octahedral manganese(II), chromium(III) and iron(III) (all are paramagnetic metal atoms with a symmetrical electronic configuration) are preferred as metals complexed by the ligands of formula I; gadolinium(III) is most preferred due to the fact that it has the highest paramagnetism, low toxicity, when complexed to a suitable ligand, and high lability of coordinated water.

The metal-chelating ligands of the present invention can be complexed with a lanthanide (atomic number 58 to 71) and used as chemical shift agents in magnetic resonance imaging or in magnetic resonance in vivo spectroscopy.

While the above-described uses for the metal-chelating ligands of the present invention are preferred, those working in the diagnostic arts will appreciate that the ligands can also be complexed with the appropriate metals and used as contrast agents in x-ray imaging, radionuclide imaging and ultrasound imaging.

### Use in Imaging

To use the ligands of this invention for imaging, they must first be complexed with the appropriate metal. This can be accomplished by methodology known in the art. For example, the metal can be added to water in the form of an oxide or in the form of a halide or acetate and treated with an equimolar amount of a ligand of the present invention. The ligand can be added as an aqueous solution or suspension. Dilute acid or base can be added (if needed) to maintain a neutral pH. Heating at temperatures as high as 100°C for periods up to four hours is sometimes required, depending on the metal and the chelator, and their concentrations.

Pharmaceutically acceptable salts of the metal complexes of the ligands of this invention are also useful as imaging agents. They can be prepared by using a base (e.g., an alkali metal hydroxide, meglumine or arginine) to neutralize the above-prepared metal complexes while they are still in solution. Some of the metal complexes are formally uncharged and do not need cations as counterions. Such neutral complexes are preferred as intravenously administered x-ray and NMR imaging agents over charged complexes because they provide solutions of greater physiologic tolerance due to their lower osmolality.

Sterile aqueous solutions of the chelate complexes can be administered to mammals (e.g., humans) orally, intrathecally and especially intravenously in concentrations of 0.003 to 1.0 molar. For example, for the visualization of brain lesions in canines using magnetic resonance imaging, a gadolinium complex of a ligand of formula Ia can be administered intravenously at a dose of 0.05 to 0.5 millimoles of the complex per kilogram of animal body weight, preferably at a dose of 0.1 to 0.3 millimole/kilogram. For visualization of the kidneys, the dose is preferably 0.05 to 0.25 millimoles/kilogram. For visualization of the heart, the dose is preferably 0.25 to 1.0 millimoles/ kilogram. The pH of the formulation will be between about 6.0 and 8.0, preferably between about 6.5 and 7.5. Physiologically acceptable buffers (e.g., tris(hydroxymethyl)aminomethane) and other physiologically acceptable additives (e.g., stabilizers such as parabens) can be present.

It is also advantageous to employ dual scavenging excipients such as those described in a copending application U. S. Ser No. 682,487 filed April 9, 1991 entitled "DUAL FUNCTIONING EXCIPIENT FOR METAL CHELATE CONTRAST AGENTS". Those excipients have the general formula

Xₘ[X'(L')]ₙ

wherein X and X' are independently Ca or Zn, L' is an organic ligand which may be different than or the same as the ligand employed to complex the metal and m and n are independently 1, 2 or 3.

### Use of Radiotherapy or Imaging Where the Metal-Chelate-Complex is Bound to a Biomolecule

The bifunctional metal-chelating ligands can bind to a monoclonal antibody or a fragment thereof for use in radiotherapy. Monoclonal antibodies are useful in that they can be used to target radionuclides to cancer or tumor sites with great specificity. The compounds of this invention wherein R₁ is other than hydrogen are then linked to monoclonal antibodies or fragments thereof.

The methods of linking the bifunctional chelate to the antibody or antibody fragment are known in the art (Brechbiel, same reference as referred to hereinabove) and will depend primarily on the particular bifunctional chelate and secondarily on the antibody or fragment thereof. For example, when the formula Ia compound is R₁ = H, R₂ = -NCS or 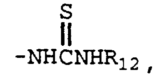 one reacts 10 µL of a 5.0 mM aqueous solution of the formula Ia chelator with 0.5 mL of a 5.0 mg/mL monoclonal antibody (B72.3 purchaseable from Damon Biotech Corporation) in 50 mM Hepes buffer at pH 8.5. 16 µL of 1.5M aqueous triethylamine is added. After 2 hours reaction time, the monoclonal antibody is purified by dialysis. This procedure provides between 1 and 2 formula Ia chelator molecules bound to each monoclonal antibody. Radioactive metal ion (for example ⁹⁰Y) can then be added to the monoclonal antibody-bound chelator by methods known in the art. For example, ⁹⁰Y as the ⁹⁰Y(III)(acetate)₃(H₂O)₄ (approximate formula in aqueous solution) can be reacted with the monoclonal antibody-bound chelate in solutions where the concentration of each is between 10⁻⁵ and 10⁻⁷ and the pH is 6. Dialysis against citrate is then used to purify the product.

An alternative, and preferred method follows that described above, but substitutes the metalchelate complex for the chelating ligand. To use this method the metal chelate complex is first made by reacting Metal-oxide, -halide, nitrate -acetate, or the like with formula Ia chelator. For the chelator described above the acetate of ⁹⁰Y at <10⁻⁶M is reacted with the chelator at about 10⁻³ at pH 6, the chelate complex is purified by ion exchange or reverse phase HPLC choromatography, and then reacted with the monoclonal antibody described above for the chelator. The bifunctional, metal-containing, linked antibody is used in the following manner. A human or animal with a tumor to which the monoclonal antibody is specific is injected intravenously, subcutaneously, intraparetoneally or intralymphatically for example, with an aqueous solution of the ⁹⁰Y-formula Ia chelator-monoclonal antibody compound. This allows the radioactive metal ion to be directed to the tumor for which it is intended. The intravenous dosaged used is 0.1 to 0.4 millicurie per kilogram of body weight.

Preferred embodiments for when the compounds are linked to a protein are when R₁ and/or R₂ = NCS is reacted with protein to produce the protein conjugate. Preferred proteins are those in serum, wherein the R₁ and/or R₂ = -NCS compound is directly injected.

It is understood that other functional groups known in the art can be used to link the bifunctional metal-chelating ligands of this invention to monoclonal antibodies or fragments thereof.

R₁ and R₂ are each 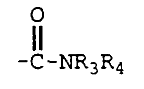 and R₃ in each is hydroxyalkyl in a preferred embodiment for forming a Gd(III) chelate useful in general purpose magnetic resonance imaging. The most preferred embodiments for forming a Gd(III) chelate are when the R₃ groups are each 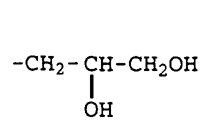 or -CH(CH₂OH)₂, especially 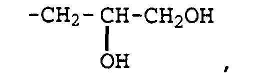 and the R₄ groups are each hydrogen.

The present invention also includes multimeric forms of the compounds of formula Ia, such as dimers, trimers, tetramers, etc. Known functional groups and technology as those discused above regarding conjugation with biomolecules are readily useable to provide such multimers. The functional groups provided onto the phenyl ring 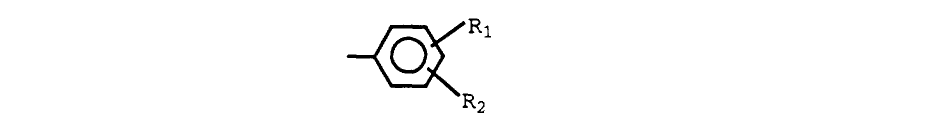 can be, for example, R₂ = NCS or 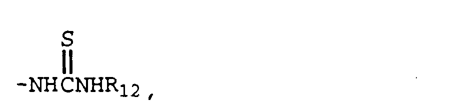 especially where R₁₂ is methyl or ethyl. Thus, exemplary multimers of formula I 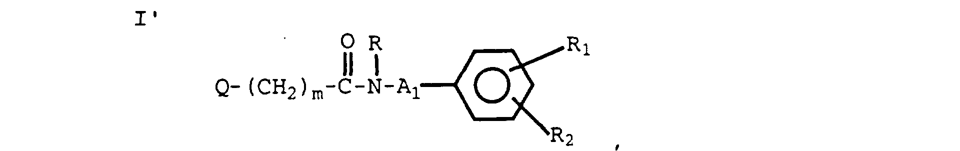 where Q is the aminocarboxylate nucleus of Ia are shown by

### Dimers

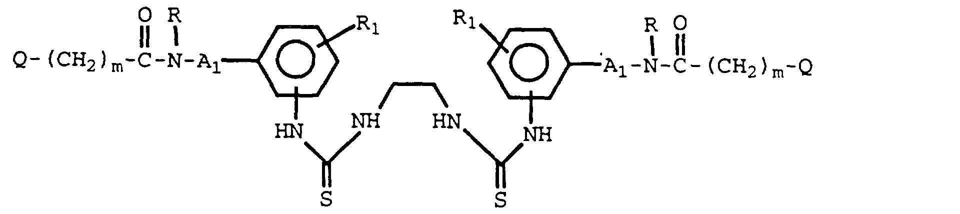

or

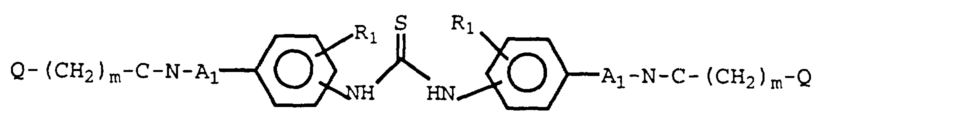

### Trimers

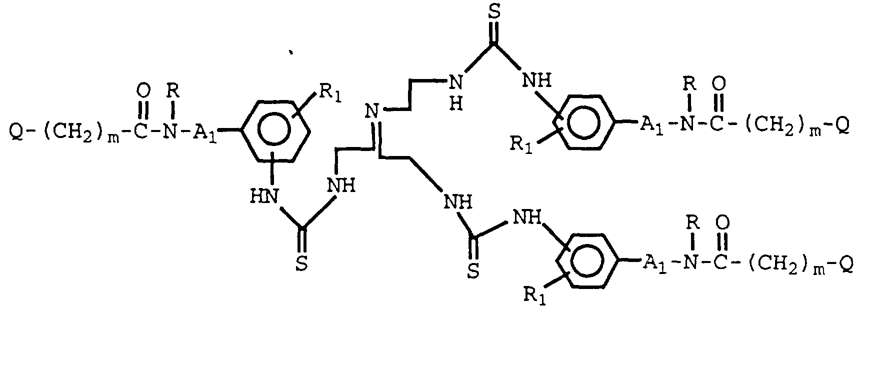

### Octamers

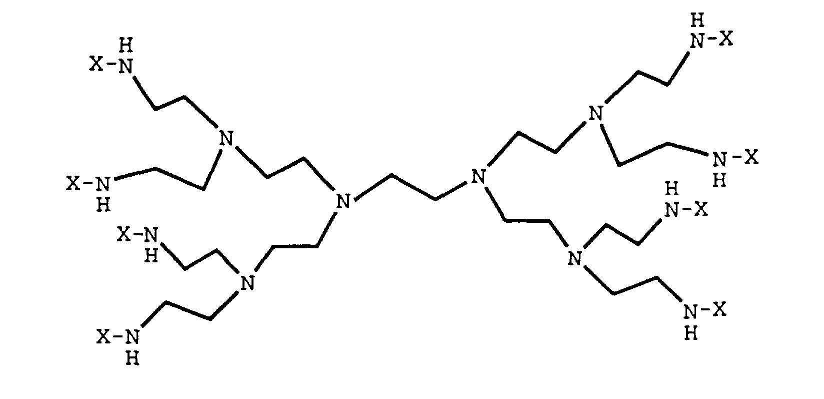

where

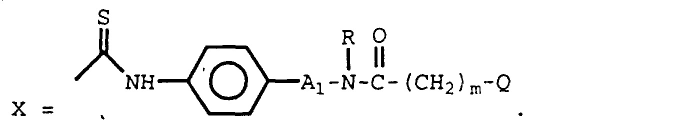

### Preparation of Formula Ia Compounds

To prepare the compounds of formula Ia, a compound of the formula 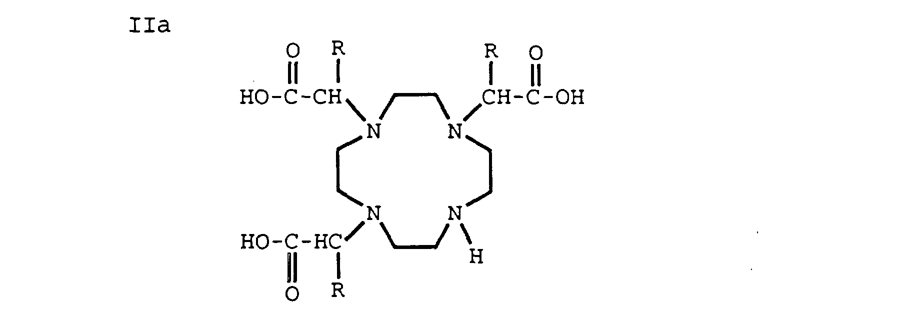 is reacted in a solvent, e.g., water, and in the presence of a base, e.g., sodium hydroxide, with a compound of the formula 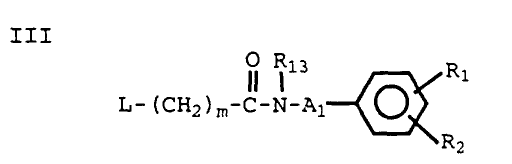 wherein L is a leaving group, such as halogen. The preparation of compounds of formula II is well known, for example, in U. S. 4,885,363 to Tweedle et al. For example, in preparing compounds of formula II, reaction of a compound of the formula 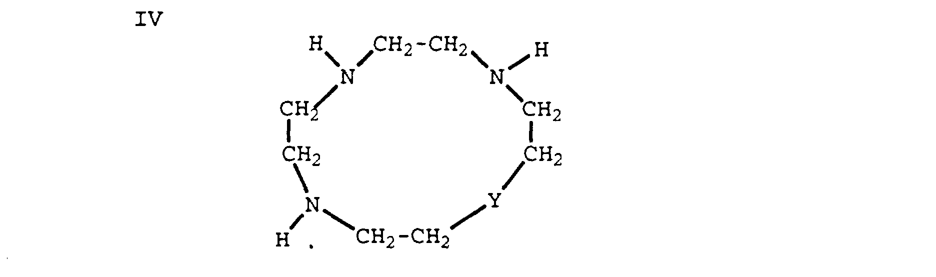 with a compound of the formula 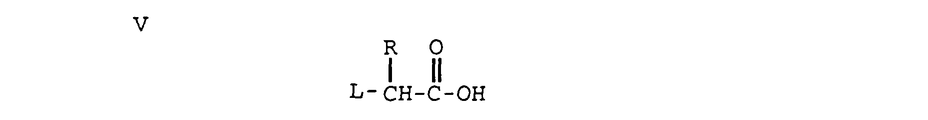 wherein L is a leaving group such as halogen is preferably carried out in water at a pH of about 8.5 to 9 and the temperature of the reaction is maintained at about 45°-55°C. Preferably, only about two equivalents of a compound of formula V are initially used in the reaction; an additional equivalent of the compound of formula V is added in portions starting about 2 to 3 hours after the reaction begins. Total reaction time will preferably be about 8 to 24 hours. The desired trisubstituted product can be separated from the reaction mixture, which includes the mono-, di-, tri- and tetrasubstituted derivatives, by techniques recognized in the art including selective precipitation, chromatography and crystallization.

A preferred preparation of the compounds of formula II wherein R is hydrogen is to react 1,4,7,10-tetraazacyclododecane, known in the art, with dimethylformamidedimethylacetal in the presence of benzene to yield 1,4,7,10-tetraazatricyclo-[5.5,1.0]tridecane. This "tricyclic" compound is reacted with an ethanol/water mixture to yield 1-formyl-1,4,7,10-tetraazacyclododecane. This formyl compound is then reacted with t-butyl bromoacetate to yield 1-formyl, 4,7,10-triscarboxymethyl-1,4,7,10-tetraazacyclododecane, tris-t-butylester. Finally, the ester groups are removed in the presence of strong acid, such as sulfuric acid, to yield a compound of formula 'II wherein R is hydrogen. The most preferred methods are included in Dischino, et al., Inorg. Chem., 30, 1265, 1991.

Compounds of formula III wherein R₁ and R₂ are each 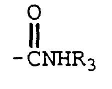 and X is -NH- are prepared by first reacting a compound of the formula 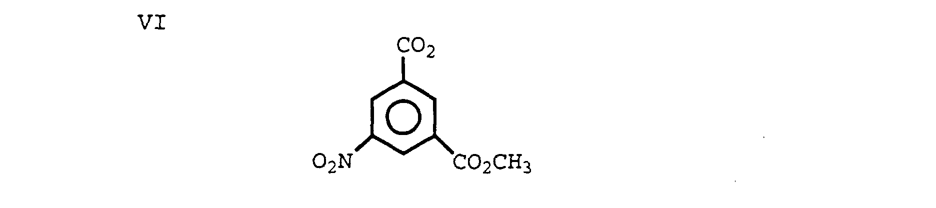 in a solvent, e.g., methanol, with a compound of the formula

VII H₂NR₃

to provide the intermediate

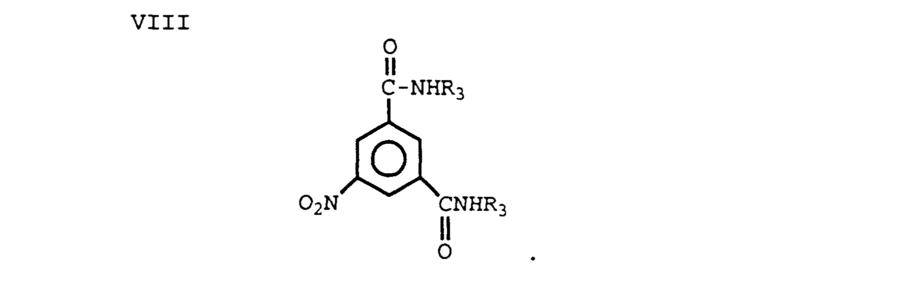

Compounds of formula VIII can thereafter be reduced, e.g., with hydrogen in the presence of a palladium on carbon catalyst, to provide

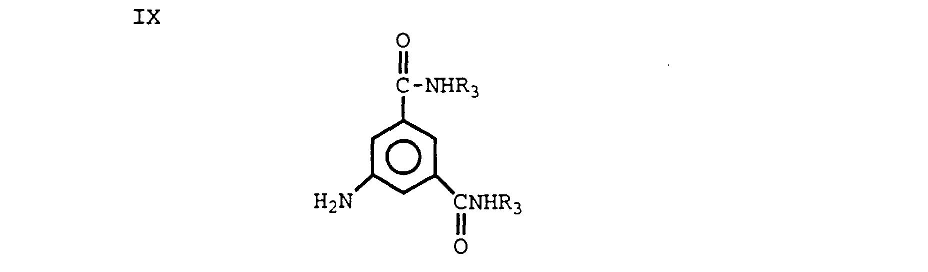

Reaction of compound IX with a compound of the formula 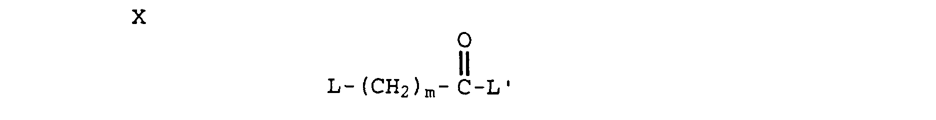 wherein L and L' are the same or different leaving groups, e.g., halogens, in a solvent, e.g., dimethylacetamide, provides the compounds of the formula 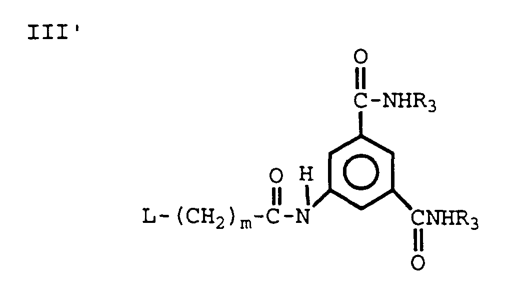 that is, compounds of formula III where R₁ and R₂ are each 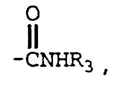 A₁ is a single bond, and X is NH.

In the event that the R₃ group in intermediate VII contains hydroxyalkyl moieties, the hydroxy groups are converted to acetyloxy groups in the reaction of compounds VI and VII. Thus, acetyloxy groups in intermediate III' are converted to hydroxy groups by known treatment, for example, with sodium methoxide in a solvent, e.g., methanol. For example, if the compound of formula VII is 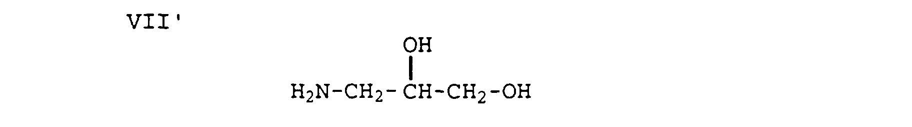 reaction as described above with compound VI provides 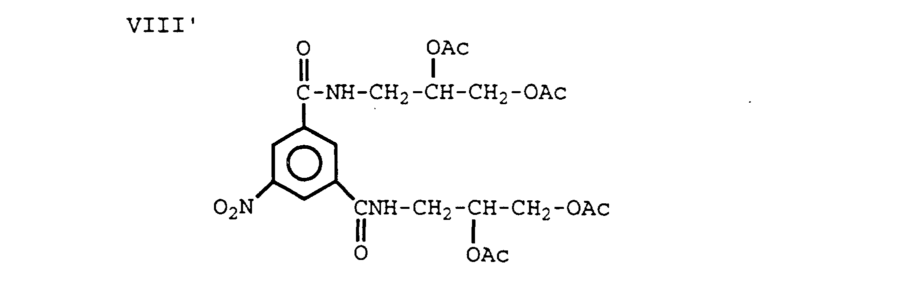 wherein Ac is acetyl. Following reduction to the corresponding aniline and thereafter reaction with compound X, the corresponding intermediates of III' are converted to their hydroxyalkyl counterparts as described above.

Compounds of formula III wherein A₁ is -CH₂-, X is 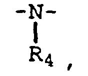 p is zero, Y is =O, L is chloro and R₁ and R₂ are each 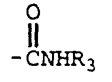 are prepared by first reacting a compound of the formula 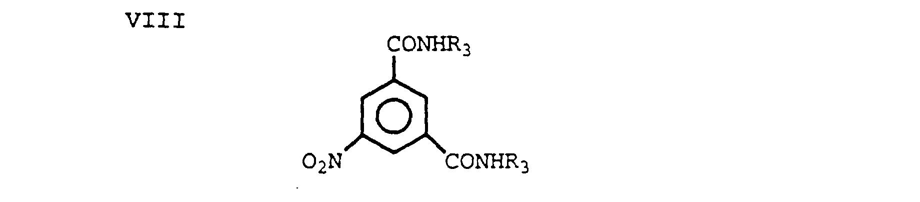 with gaseous hydrqgen in the presence of a catalyst such as palladium on carbon in dilute mineral acid to provide the aniline

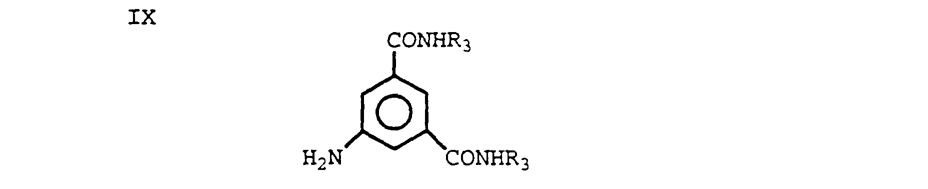

The aniline is diazotized with nitrous acid in acidic medium and then treated with sodium cyanide to obtain

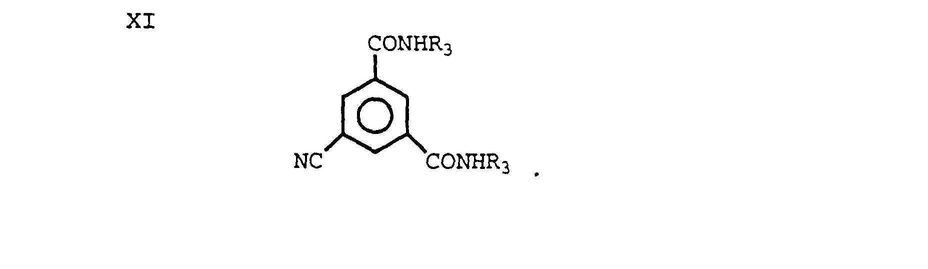

Reduction of the nitrile XI in the presence of a platinum catalyst with gaseous hydrogen at low pressure, for example 3 atmospheres, affords

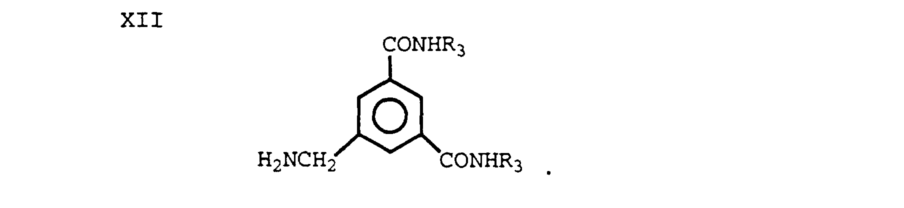

Reaction of XII with a compound of formula 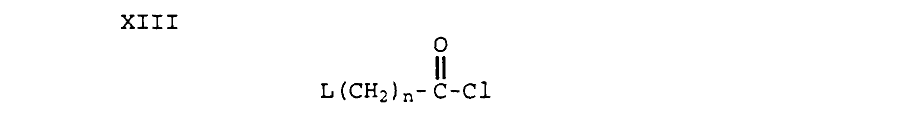 wherein L is a leaving group, for example chlorine, provides compounds of the formula

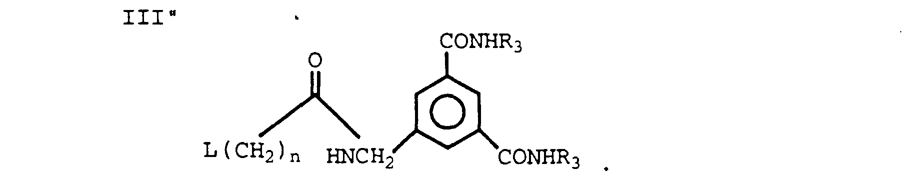

If R₄ is other than hydrogen, for example methyl, the compound of the formula XII is treated with an aldehyde R₈CHO, for example formaldehyde, wherein R₈ is H, under reducing conditions, for example with sodium borohydride, to obtain 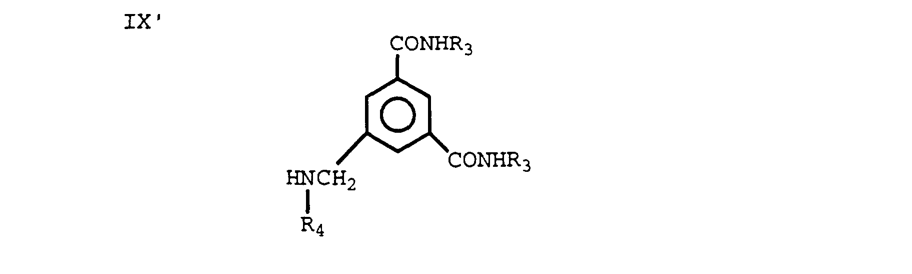 wherein R₄ is methyl. Reaction of IX' with the chloride of formula XIII will provide the desired intermediate of the formula

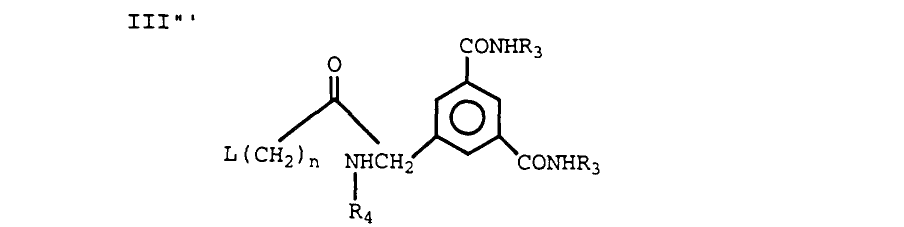

Compounds of formula Ia wherein X is -CH₂- or a single bond, p is 1, Y is -OH, m is 1 and R₁ and R₂ are each -CONHR₃ are prepared by reacting the compound of formula II with an epoxide of the formula 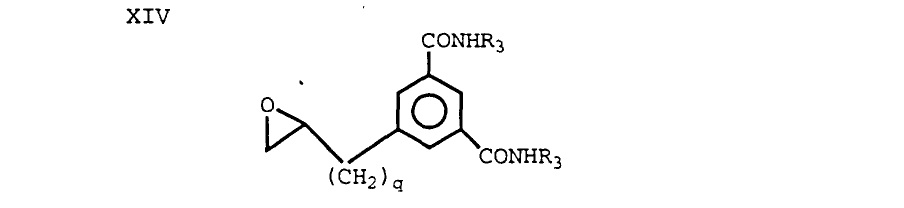 in aqueous alkaline media wherein q 1 or 0. If q is 1, the reaction yields compounds of formula I wherein X is CH₂ and if q is zero, the reaction yields compounds of formula I wherein X is a single bond.

Compounds of formula XIV are prepared by diazotizing the amines of formula IX to obtain the diazonium salts of formula 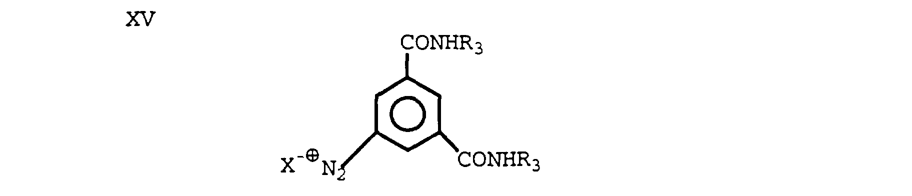 wherein X⁻ is a nonnucleophilic radical such as BF₄⁻. Treatment of compounds of formula XV with tetraallyl tin and a palladium acetate catalyst will afford

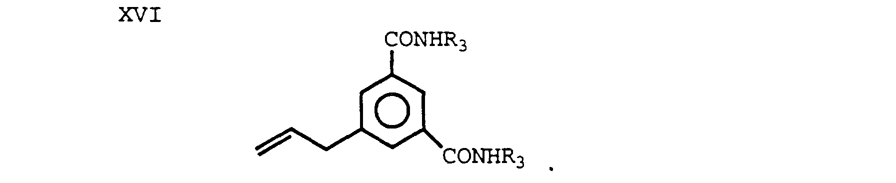

Epoxidation of XVI using a peracid, such as m-chloroperoxybenzoic acid, will yield the compound of the formula 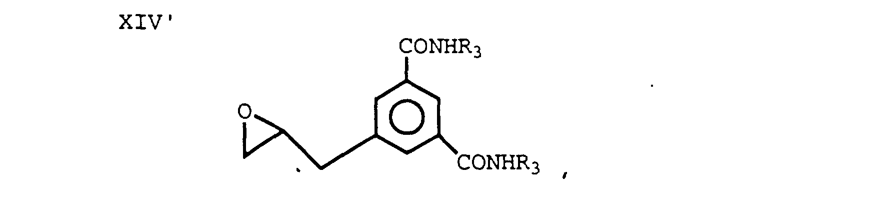 that is, compounds of formula XIV, wherein q is 1. Alternatively, treatment of the diazonium salt of formula XV with tributyl-vinyl tin 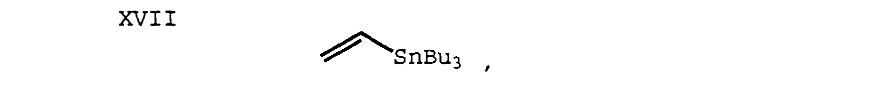 and a palladium acetate catalyst, will afford the compound of formula

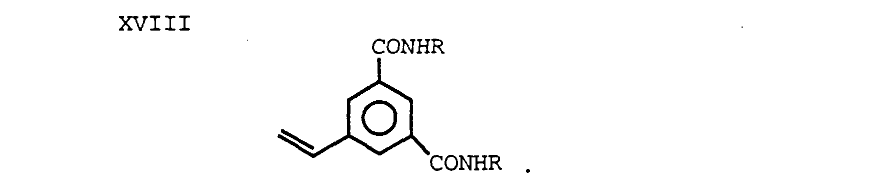

Epoxidation of XVIII using a peracid or H₂O₂ in the presence of benzonitrile or any of the other epoxidation methods known to those well versed in the art will provide the compound of formula 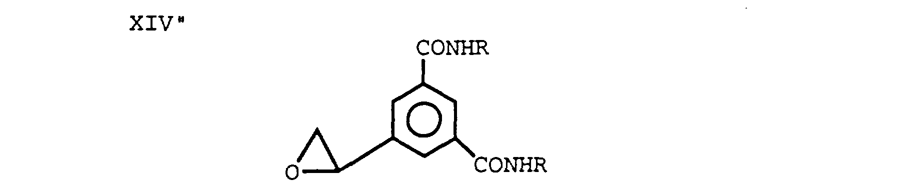 that is, compounds of formula XIV wherin q is zero.

The invention will now be further described by the following examples.

### Example 1

### 10-[2-[[3,5-Bis[[(2,3-dihydroxypropyl)amino]-carbonyl]phenyl]amino]-2-oxoethyl]-1,4,7,10-tetraazacyclododecane-1,4,7-triacetic acid, monogadolinium complex

### A. N,N'-Bis[2,3-bis(acetyloxy)propyl]-5-nitro-1,3 -benzenedicarboxamide

To a solution of dimethyl-5-nitroisophthalate (23.9 g, 100 mmol) in methanol (300 mL) was added 1-amino-2,3-propanediol (20.2 g) and the mixture was refluxed for 48 hours. Methanol was removed in vacuo, the residue dissolved in pyridine (150 mL) and then treated with acetic anhydride (80 mL) at room temperature for 16 hours. Excess of acetic anhydride was decomposed by adding water (50 mL) to the reaction mixture. The solvents were removed in vacuo, the residue dissolved in ethyl acetate (400 mL) and washed with water (2 x 100 mL), 10% hydrochloric acid (200 mL) and finally with brine (100 mL). The ethyl acetate layer was dried and removal of the solvent afforded the title A nitro-bis-amide (51.8 g), as a light yellow viscous syrupy material. This was used directly without further purification in the next step.

### B. 5-Amino-N,N'-Bis[2,3-bis(acetyloxy)propyl]-1.3-benzenedicarboxamide

A solution of the title A nitrobisamide (31.5 g, 60 mmol) in methanol (180 mL) was hydrogenated over 10% palladium on carbon (300 mg) for a period of 3 hours. The catalyst was filtered off and the solvent removed in vacuo to afford pure title B aniline (28.6 g), as a viscous syrupy material. This was used directly without further purification in the next step.

### C. N,N'-Bis[2,3-bis(acetyloxy)propyl]-5-N-[(chloroacetyl)amino]-1,3-benzenedicarboxamide

The title B aniline was dissolved in dimethylacetamide (150 mL) and treated with chloroacetyl chloride (11.28 g, 100 mmol) dropwise over a period of 20 minutes. The solution was stirred for 3 hours and dimethylacetamide removed in vacuo. The residue that resulted was dissolved in ethyl acetate (300 mL), washed with water (150 mL), 10% aqueous sodium bicarbonate (150 mL), and finally with water (150 mL). The ethyl acetate layer was dried and removal of the solvent afforded the crude chloroacetanilide (32.0 g). The crude material was purified by column chromatography over silica gel to obtain the title C compound (26.3 g), as a colorless glassy solid. An analytical sample was prepared by crystallizing 1.00 g of the glassy solid from ethyl acetate (5 mL) and hexane (1.0 mL).
Elemental Analysis calc'd for C₂₄H₃₀N₃ClO₁₁:
C, 50.40; H, 5.29; N, 7.35; Cl, 6.20; O, 30.77;
Found: C, 50.28; H, 5.15; N, 7.11; Cl, 6.25.

### D. 5-[(Chloroacetyl)amino-N,N'-bis[2,3-dihydroxypropyl)-1,3-benzenedicarboxamide

A solution of the title C compound (25.6 g, 45 mmol) in methanol (200 mL) was treated with sodium methoxide (20 mmol) and the solution was stirred at 0° for 30 minutes. The pH of the reaction mixture was adjusted to 7 by adding Dowex 50 (H⁺) resin, the resin filtered off and the methanol removed in vacuo to afford pure title D compound as a colorless glassy solid (16.8 g). This material was directly used in the next step without further purification.

### E. 10-[2-[[3,5-Bis[[(2,3-dihydroxypropyl)amino]-carbonyl]phenyl]amino]2-oxoethyl]1,4,7,10-tetraazacyclododecane-1,4,7-triacetic acid

A solution of DO3A sulfate (DO3A = 1,4,7,-triscarboxymethyl-1,4,7,10-tetraazacyclododecane prepared in U. S. 4,885,363 to Tweedle et al.) (12.0 g, 27 mmol) was made in water (80 mL) and the pH of the solution was adjusted to 9.8 by adding 5 M sodium hydroxide. While maintaining the pH of the solution at 9.8, a solution of the title D compound (16.4 g, 40.6 mmol) in water (50 mL) was slowly added to the DO3A solution at 80° over a period of 45 minutes. At the end of 17 hours, the reaction mixture was cooled to room temperature, the pH lowered to 3.5 by adding IN hydrochloric acid and the solution was desalted by cation exchange chromatography. Further purification by anion exchange chromatography afforded the title E compound as the triethylammonium salt (19.9 g). The triethylamine salt was dissolved (6.00 g) in water (1 L), applied to an anion exchange column and then eluted with 50 mM formic acid to obtain the desired title E compound HAA-DO3A (4.9 g). IR: 3400 (OH); 3115 (NH); 1631 (COOH and ArCONH) cm⁻¹. Mass Spectrum: 714 (M+H)⁺; 712 (M-H)⁻.
Elemental Analysis calc'd for C₃₀H₄₇N₇O₁₃·0.38H₂O:
C, 50.01; H, 6.68; N, 13.61; O, 29.71;
Found: C, 49.91; H, 6.97; N, 13.42; H₂O, 0.95.

### F. 10-[2-[[3,5-Bis[[(2,3-dihydroxypropyl)amino]-carbonyl]phenyl]amino]-2-oxoethyl]-1,4,7,10-tetraazacyclododecane-1,4,7-triacetic acid, monogadolinium salt

To a solution of the title E triethylammonium salt (19.00 g, 18.7 mmol) in water (80 mL) at pH 4.72 was added a solution of Gd(OAc)₃ · 4H₂O (9.83 g, 24 mmol) in water (80 mL) and the reaction mixture was stirred at room temperature for 12 hours. The reaction mixture was subjected to low pressure reversed phase column chromatography over the nonionic resin HP-20 to obtain the title compound as a colorless glassy solid (17.5 g). The pure product (17.00 g) was crystallized from hot methanol (300 mL) to afford Gd(HAA-DO3A) as colorless needles of >99.9% purity. This sample was redissolved in water (200 mL), the solvent removed, and the sample dried in vacuo (1 mm) for four days at 80°. Mass Spectrum: 869 (M+H)⁺, 867 (M-H)⁻¹.
Elemental Analysis: C₃₀H₄₄N₇O₁₃ · 0.36 H₂O:
C, 41.20; H, 5.15; N, 11.21; O, 24.45
Found: C, 40.96; H, 5.07; N, 10.93; H₂O, 0.75.

### Example 2

### 10-[2-[[3,5-Bis-[[[2-hydroxy-1-(hydroxymethyl)ethyl]-amino]carbonyl]phenylamino]2-oxoethyl]1,4,7,10-tetraazacyclododecane-1,4,7-triacetic acid

### A. N,N'-bis[2-(Acetyloxy)-1-[(acetyloxy)methyl]-ethyl] -5-nitro-1,3-benzenedicarboxamide

To a solution of dimethyl-5-nitroisophthalate (14.0 g, 58 mmol) in methanol (150 mL) was added 2-amino-1,3-propanediol (16.5 g, 181 mmol) and the mixture was refluxed for 48 hours. The reaction mixture was cooled to room temperature and the crystalline solid that separated out filtered and dried to obtain the bis amide (19.5 g). A solution of the bis-amide (19.0 g) in pyridine (75 mL) was treated with acetic anhydride (40 mL) at room temperature for 16 hours. Excess of acetic anhydride was decomposed by adding water (50 mL) to the reaction mixture. The solvents were removed in vacuo, the residue dissolved in ethyl acetate (400 mL), and the solution washed with water (2 x 100 mL), 10% hydrogen chloride (200 mL) and finally with brine (100 mL). The ethyl acetate layer was dried and removal of the solvent afforded pure title A nitrobisamide (23.6 g) as a colorless solid, after crystallization from acetone and hexane, m.p. 105-107°C.

### B. 5-Amino-N,N'-bis[2-(acetyloxy)-1-[(acetyloxy)-methyl]ethyl]-1,3-benzenedicarboxamide

A solution of the title A compound (18.0 g, 34 mmol) in methanol (180 mL) was hydrogenated over palladium on carbon (0.5 g) for a period of 3 hours. The catalyst was filtered off and the solvent removed in vacuo to afford pure title B aniline (16.6 g) after crystallization from acetone and hexane, m.p. 152-154°C.
Elemental Analysis calc'd for C₂₄H₃₀N₃ClO₁₁:
C, 50.40; H, 5.29; N, 7.35; Cl, 6.20;
Found: C, 50.64; H, 5.20; N, 7.22; Cl, 6.57.

### C. N,N'-Bis[2-(acetyloxy)-1-[(acetyloxy)methyl]-ethyl]-5-(chloroacetyl)amino-1,3-benzene-dicarboxamide

The title B compound (17.0 g, 34 mmol) was dissolved in dimethylacetamide (150 mL) and treated with chloroacetyl chloride (7.52 g, 64 mmol) dropwise over a period of 20 minutes. The solution was stirred for 3 hours and dimethylacetamide was then removed in vacuo. The residue that resulted was dissolved in ethyl acetate (300 mL), washed with water (150 mL), aqueous sodium bicarbonate (10%, 150 mL), and finally with water (150 mL). The ethyl acetate layer was dried and the solvent removed to obtain the crude chloroacetyl-anilide (18.5 g). This material was crystallized from ethyl acetate and hexane to afford pure title A compound (16.8 g), m.p. 135-137°C. Mass Spectrum: 572 (M+H)⁺; 570 (M-H)⁻.
Elemental Analysis calc'd for C₂₄H₃₀N₃ClO₁₁:
C, 50.40; H, 5.29; N, 7.35; Cl, 6.20; O, 30.77;
Found: C, 50.64; H, 5.20; N, 7.22; Cl, 6.57.

### D. 5-[(Chloroacetyl)amino]-N,N'-bis[2-hydroxy-1-(hydroxymethyl)ethyl]-1,3-benzenedicarboxamide

A solution of the title C compound (16.0 g, 28 mmol) in methanol (200 mL) was treated with sodium methoxide (10 mmol) and the solution was stirred at 0° for 30 minutes. The precipitated solid was filtered and dried to afford pure title D compound as a colorless glassy solid (10.8 g), m.p. 222-224°. Mass Spectrum: m/z 404 (M+H)⁺.
Elemental analysis calc'd for C₁₆H₂₂N₃ClO₇:
C, 47.59; H, 5.49; N, 10.41; C1,8.78; O, 27.73;
Found: C, 47.66; H, 5.55; N, 9.98; Cl, 8.88.

### E. 10-[2-[[3,5-Bis-[[[2-hydroxy-1-(hydroxy-methyl)ethyl]amino]carbonyl]phenylamino]2-oxoethyl]-1,4,7,10-tetraazacyclododecane-1,4,7-triacetic acid

A solution of DO3A sulfate (6.0 g, 13.5 mmol) was made in water and the pH of the solution was adjusted to 9.8 by adding 5 M sodium hydroxide. While maintaining the pH of the solution at 9.8, solid N,N'-bis[2-hydroxy-1-[(hydroxy)methyl]ethyl]-5-N-(chloroacetyl)aminobenzene-1,3-dicarboxamide (8.2 g, 20.4 mmol) was added in small portions to the DO3A solution at 80° over a period of 45 minutes. At the end of 20 hours, the reaction mixture was cooled to room temperature, the pH lowered to 3.5 by adding IN hydrochloric acid and the solution was desalted by cation exchange column chromatography. Further purification by anion exchange column chromatography afforded the title E compound as the corresponding triethylammonium salt (5.2 g). The triethylammonium salt (5.2 g) was dissolved in water (1 L) and applied to an anion exchange column and eluted with 50 mM formic acid to obtain the pure title compound HAS-DO3A (4.4 g), as a colorless glassy solid. Mass Spectrum: 714 (M+H)⁺; 712 (M-H)-.
Elemental analysis calc'd for C₃₀H₄₇N₇O₁₃:
C, 50.48; H, 6.64; N, 13.74; O, 29.14;
Found: C, 50.34; H, 6.83; N, 13.54.

The Gd complex of this ligand was prepared by the same method used for the compound in Example 1.
Elemental Analysis for C₃₀H₄₄N₇O₁₃Gd 3.48 H₂O:
C, 38.72; H, 5.52; N, 10.53; O, 28.33;
Found: C, 39.01; H, 5.37; N, 10.26.

### Example 3

### 10-[2-[[3,5-Bis[[(2-methylbutyl)amino]carbonyl]-phenyl]amino]2-oxoethyl]1,4,7,10-tetraazacyclododecane-1,4,7-triacetic acid, monogadolinium complex

### A. N,N'-Bis[(2-methylbutyl)amino]-5-nitro-1,3-benzenedicarboxamide

To a solution of dimethyl-5-nitro-isophthalate (14.0 g, 50 mmol) in methanol was added 2-methylbutylamine (12.5 g, 150 mmol) and the mixture was refluxed for 48 hours. Methanol was removed in vacuo, the residue dissolved in ethyl acetate (200 mL) and washed with 10% hydrochloric acid (200 mL), 10% aqueous sodium bicarbonte solution (20 mL) and finally with water (100 mL). The ethyl acetate layer was dried and removal of the solvent afforded the desired compound. This was crystallized from ethyl acetate and hexane to afford the the title A compound as colorless needles (19.6 g), m.p. 147-148°C.

### B. 5-Amino-N,N'-bis[(2-methylbutyl)amino]-1,3-benzenedicarboxamide

A solution of the title A compound (17.45 g, 50 mmol) in methanol (180 mL) was hydrogenated over 10% palladium on carbon (500 mg) for a period of 3 hours. The catalyst was filterd off and the solvent removed in vacuo to afford the title aniline as a colorless solid. This was crystallized from acetone and hexane to afford the title B compound as colorless needles (15.8 g), m.p. 170-172°C.

### C. 5-[(Chloroacetyl)amino]-N,N-bis[(2-methylbutyl)amino]1,3-benzenedicarboxamide

A solution of the title B compound (11.48 g, 36 mmol) in dimethylacetamide (200 mL) was treated with chloroacetyl chloride (5.6 g, 50 mmol) dropwise over a period of 20 minutes. The solution was stirred for 3 hours and dimethylacetamide removed in vacuo. The residue that resulted was dissolved in ethyl acetate (200 mL), washed with water (100 mL), 10% aqueous sodium bicarboante (100 mL) and finally with water (100 mL). The ethyl acetate layer was dried and removal of the solvent afforded the crude chloroacetanilide (12.8 g). This was crystallized from ethyl acetate and hexane to afford the title C compound as colorless needles (11.2 g), m.p. 160-162°C.

### D. 10-[2-[[3,5-Bis[[(2-methylbutyl)amino)-carbonyl]phenyl]amino]2-oxoethyl]1,4,7,10-tetraazacyclododecane-1,4,7-triacetic acid

A solution of DO3A sulfate (6.0 g, 13.5 mmol) was made in water (100 mL) and the pH of the solution was adjusted to 9.8 by adding 5 M sodium hydroxide. While maintaining the pH of the solution at 9.8, a solution of the n-chloroacetyl anilide (8.2 g, 27 mmol) in ethanol (100 mL) was slowly added to the DO3A solution at 80° over a period of 1 hour. At the end of 17 hours, the reaction mixture was cooled to room temperature, the pH lowered to 3.5 by adding 1N hydrochloric acid and the solution was desalted by cation exchange chromatography. Further purification by anion exchange chromatography afforded the title compound as the triethyl ammonium salt (2.8 g). The triethyl ammonium salt was dissolved in water, applied to an anion exchange column and then eluted with 50 mM formic acid to obtain the desired AAA-DO3A (2.2 g). A small amount of an impurity present in this sample was further removed by a reverse phase CHP-20 column chromatography to afford the title compound as a colorless glassy solid (1.8 g). Mass Spectrum: 706 (M+H)⁺; 704 (M-H)⁻.
Elemental analysis calc'd for C₃₄H₅₅N₇O₉·1.7 H₂O:
C, 55.45; H, 7.99; N, 13.31; O, 23.24;
Found: C, 55.85; H, 8.37; N, 13.19; H₂O: 4.15.

The Gd complex of this ligand was prepared by the same method as used for the compound of Example 1.
Elemental anal. calc'd for C₃₄H₅₂N₇O₉Gd, 6.44 H₂O:
C, 41.84; H, 6.70; N, 10.04; O, 26.31;
Found: C, 41.80; H, 6.65; N, 10.28.

The T₁ relaxivity was measured for nine prior art gadolinium complexes (1-9) as compared to novel gadolinium complexes using the ligands of Examples 1, 2 and 3 (#10, 11 and 12, respectively, in the Table below). Relaxivity was measured on an IBM Minispec spin analyzer operating at 20 MHz and 39±1°C. Aqueous solutions were used in 0.1-5 mM Gd concentration range.

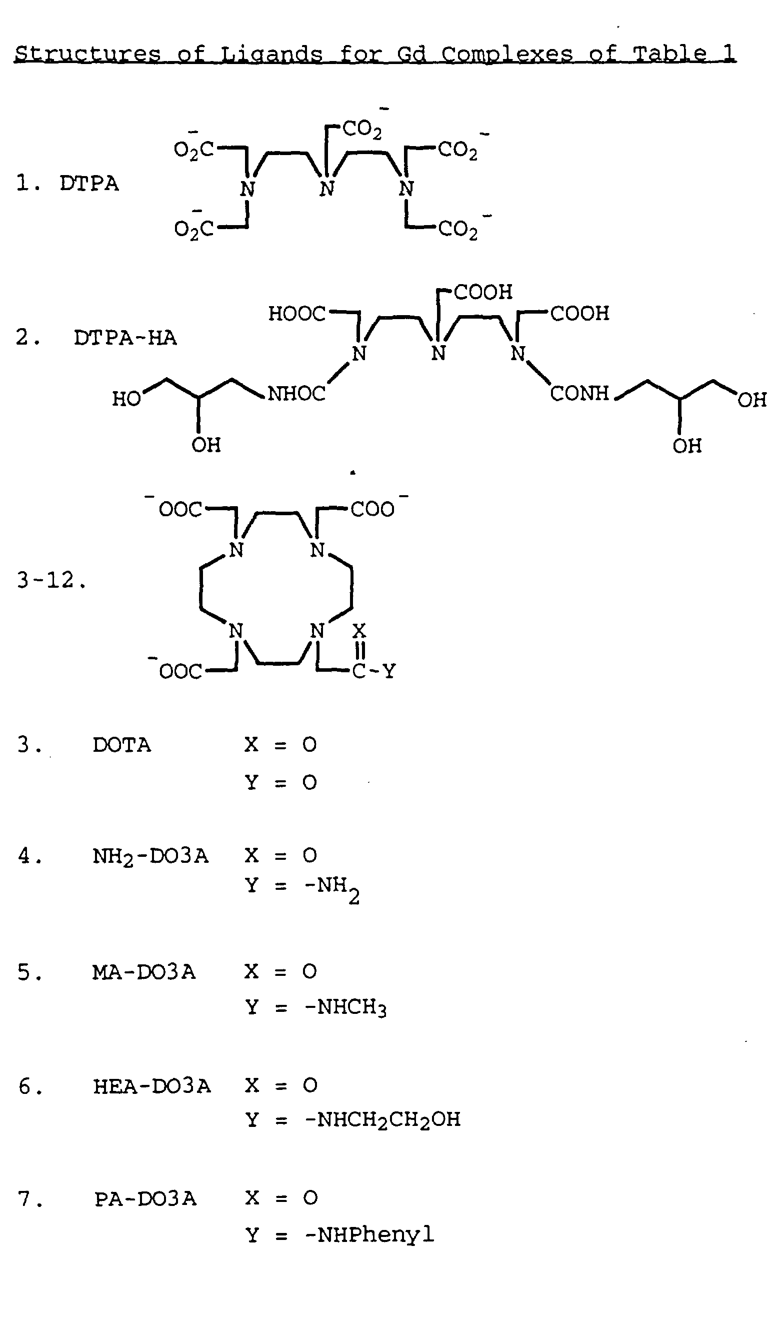

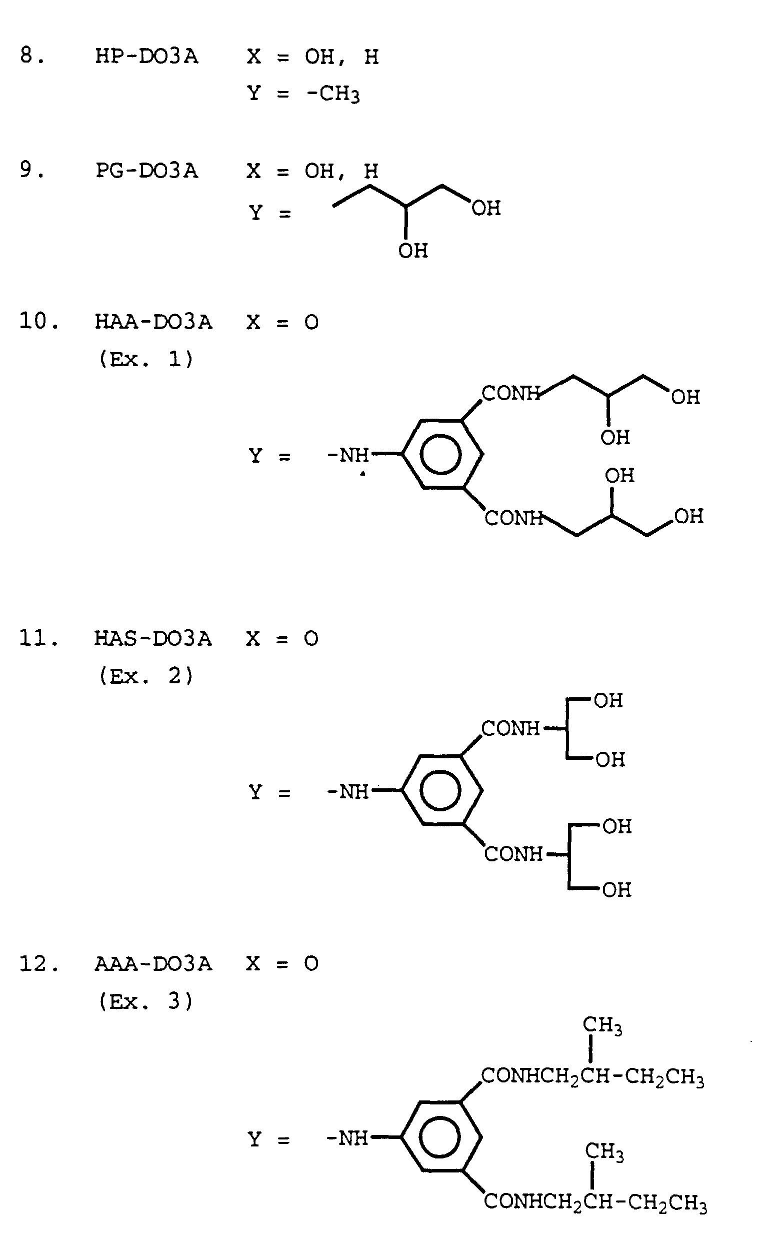

**Table 1**

| Data on Water Soluble Gd Complexes and IonsDemonstrating The Enhancement of Relaxivity by N-Hydroxy-alkyl or N-alkyl-isophthalamide Groups and by aryl groups or by hydroxyalkyl or alkylamido groups. | | |
|---|---|---|
| | Gd(L), L = | T₁ Relaxivity |
| 1. | DTPA | 3.7 |
| 2. | DTPA-HA | 4.4 |
| 3. | DOTA | 3.4 |
| 4. | NH₂-DO3A | 3.6 |
| 5. | MA-DO3A | 4.3 |
| 6. | HEA-DO3A | 4.3 |
| 7. | PA-DO3A | 4.1 |
| 8. | HP-DO3A | 3.7 |
| 9. | PG-DO3A | 3.4 |
| | | |
| 10. | HAA-DO3A (Ex. 1) | 5.8 |
| 11. | HAS-DO3A (Ex. 2) | 5.4 |
| 12. | AAA-DO3A (Ex. 3) | 5.9 |

The relaxivity is especially high only in the substituted aryl compounds, 10, 11 and 12, i.e., Gd(HAA-DO3A), Gd(HAS-DO3A) and Gd(AAA-DO3A).

One or two hydroxy groups alone do not enhance relaxivity, as can be seen from L = HP-DO3A, PG-DO3A. Alkyl or aryl substituents only slightly enhance relaxivity, as seen from MA-DO3A and PA-DO3A. Both alkyl and hydroxyalkyl substituents on the aromatic are effective at enhancing relaxivity (the hydroxyalkyls are preferred for their increased water solubility).

### Example 4

### 10-[2-[Methyl[3,5-bis[[(2-methylbutyl)amino]-carbonyl]phenyl]amino]-2-oxoethyl]-1,4,7,10-tetraazacyclododecane-1,4,7-triacetic acid, monogadolinium salt

### A. N,N'-bis(2-Methylbutyl)-5-[[(phenylmethoxy)-carbonyl]-amino]-1,3-benzenedicarboxamide

To a cooled solution of compound A from Example 3 (15.4 g, 46 mmol) in anhydrous DMA (75 ml) at O°C was added benzyl chloroformate (9.4 g, 55.2 mmol). The clear solution was stirred at 0°C for 2 hours. DMA was removed in vacuo. The residue was dissolved in EtOAc (150 ml), and was washed with aqueous NaHCO₃ solution (30 ml) and with H₂O (2 x 50 ml). The organic layer was dried over anhydrous MgSO₄ and the solvent removed to obtain the crude product as an oily liquid Recrystallization of the crude material from EtOAc/hexanes (5/1) afforded the title A product as a white solid (17.0 g), m.p. 130.5-132.5°C.
Elemental analysis calc'd for C₂₆H₃₅N₃O₄:
C, 68.85; H, 7.78; N, 9.26; O, 14.11;
Found: C, 68.64; H, 7.91; N, 9.20.

### B. N,N'-bis(2-Methylbutyl)-5-[methyl[(phenylmethoxy)carbonyl]amino]-1,3-benzenedicarboxamide

to a suspension of NaH (0.58 g, 24.2 mmol) in anhydrous THF (25 ml) was added a solution of the title A compound (10.0 g, 22 mmol) in anhydrous THF (60 ml). Mel (15.7 g, 110 mmol) was added and the reaction mixture was stirred at room temperature for 1 hour. THF was removed in vacuo. The solid was dissolved in EtOAc (150 ml) and was wshed with H₂O (2 x 50 ml), then with aqueous NaCl solution (50 ml). The EtOAc layer was dried over anhydrous MgSO₄ and the solvent was removed to obtain the title B compound.
Elemental analysis calc'd for C₂₇H₃₇N₃O₄:
C, 69.35; H, 7.98; N, 8.99; O, 13.69;
Found: C, 69.10; H, 8.03; N, 8.91.

### C. 5-(Methylamino)-N,N'-bis(2-methylbutyl)-1,3-benzenedicarboxamide

To a solution of the title B compound (13 g, 27.8 mmol) in MeOH (50 ml) was added 1,4-cyclohexadiene (20 ml) and 10% Pd/C (3.25 g). The mixture was refluxed for 0.5 hour. The solid was filtered through a celite cake and the solvent was removed to obtain the crude product. Recrystallization from hot EtOAc afforded the title C product as white crystals (5.2 g), m.p. 160.1-160.8°C.
Elemental analysis calc'd for C₁₉H₃₁N₃O₂:
C, 68.43; H, 9.37; N, 12.60;
Found: C, 68.13; H, 9.50; N, 12.57.

### D. 5-[(Chloroacetyl)methylamino]-N,N'-bis(2-methylbutyl)-1,3-benzenedicarboxamide

To a solution of the title C compound (5.2 g, 15.6 mmol) in anhydrous DMA (150 ml) was added chloroacetylchloride (2.43 g, 5.9 mmol). The solution was stirred at room temperature for 1.5 hours. The mixture was cooled. Water (20 ml) was added and the solvent removed in vacuo. The residue was dissolved in EtOAc (200 ml) and washed with aqueous NaHCO₃ solution (50 ml), then with water (2 x 50 ml). The organic layer was dried over anhydrous MgSO₄ and the solvent removed to obtain the crude product. Recrystallization from hot EtOAc afforded the title D compound as white crystals (6.0 g), m.p. 170.0-171.5°C.
Elemental analysis calc'd for C₂₁H₃₂N₃O₃Cl:
C, 61.53; H, 7.87; N, 10.25; Cl, 8.65; O, 11.71
Found: C, 61.77; H, 7.83; N, 10.39; Cl, 8.41.

### E. 10-[2-Methyl[3,5-bis[[(2-methylbutyl)amino]-carbonyl]phenyl]amino]-2-oxoethyl]-1,4,7-10-tetraazacyclododecane-1,4,7-triacetic acid

DO3A sulfate (4.35 g, 9.8 mmol) was dissolved in H₂O (100 ml) and the pH of the solution adjusted to 9.8 by adding 10 N NaOH. To this solution at 85°C was added a solution of the title D compound (5.7 g, 13.9 mmol) in EtOH (100 ml) over a period of 45 minutes. The pH was maintained at 9.8 by adding 5 N NaOH. The mixture was heated at 85°C for 44 hours. The solvents were removed in vacuo. The solid was dissolved in H₂O (300 ml) and EtOAc (100 ml) and the cloudy solution was stirred at 85°C for 2 hours until the mixture turned clear. The two layers were separated. The aqueous layer (pH 7) which contained the crude product was applied to a 300 ml column of CHP-20 resin using EtOH/H₂O (0-10%) as an eluent. The fractions containing the desired compound were combined and removal of the solvent afforded the title E product as a monosodium salt (2.9 g).

### F. 10-[2-[Methyl[[3,5-bis[[(2-methylbutyl)amino]-carbonyl]phenyl[amino]-2-oxoethyl]-1,4,7,10-tetraazacyclododecane-1,4,7-triacetic acid, monogadolinium salt

The title E compound (700 mg, 0.97 mmol) was dissolved in H20 (8 ml) and the pH of the solution adjusted to 4.5 by adding diluted HOAc. To this solution was added a solution of Gd(OAc)3·4H₂O (1.21 g, 1.3 mmol) in H₂O (10 ml). The mixture was stirred at 45°C for 24 hours. The solution was then applied to a 600 ml column of CHP=20 resin, using EtOH/H₂O (0-50%) as an eluent. The fractions containing the desired compound were combined and removal of the solvent afforded 770 mg of the title product.
Elemental analysis calc'd for C₃₄H₅₂N₇O₉Gd·1.10H₂O:
C, 46.42; H, 6.21; N, 11.14; Gd, 18.28; O, 16.74;
Found: C, 46.68; H, 6.35; N, 10.88.

### Example 5

### 10-[2-[[4-[[2,3-Dihydroxypropyl)amino]carbonyl]-phenyl]amino]-2-oxoethyl-1,4,7,10-tetraazacyclododecane-1,4,7-triacetic acid, monogadolinium salt

### A. N-(2,3-Diacetylpropyl)-4-carboxyamido nitrobenzene

To a solution of methyl 4-nitro benzoate (18.1 g, 100 mmol) in 200 ml of MeOH was added 3-amino-1,2-propanediol (18.2 g, 200 mmol) and the mixture was refluxed for 24 hours. The product then was directly acetylated. Methanol was removed in vacuo. The residue was dissolved in 100 ml of pyridine and 80 ml of acetic anhydride was added. The solution was stirred at room temperature for 24 hours. The solution was cooled and water was added to decompose the excess of acetic anhydride. The solvents were removed in vacuo. The residue was dissolved in EtOAc (300 ml) and it was washed with H₂O (2 x 80 ml), 10% HCl (150 ml) and finally with brine (150 ml). The organic layer was dried and removal of the solvent afforded 28.3 g of the title A compound as a yellowish solid (87.3 mmol), m.p. 101.5-102.8°.
Elemental analysis calc'd for C₁₄H₁₆N₂O₇:
C, 51.85; H, 4.97; N, 8.64;
Found: C, 51.69; H, 5.00; N, 8.58.

### B. N-(2,3-Diacetylpropyl)-4-carboxyamido aniline

A solution of the title A compound (12 g, 37 mmol) in 120 ml of EtOAc was mixed with 5% Pd/C (1.2 g). The solution was hydrogenated at 45 psi pressure until the pressure dropped down to a constant value. The solid was then filtered. The filtrate was concentrated to dryness and 10.8 g of the title B product as a foaming liquid was obtained (36.7 mmol). TLC: Silica gel, R_{f} 0.70, EtOAc, visualized by UV.

### C. 4[(Chloroacetyl)amino]-N-(2,3-dihydroxypropyl)-1-benzenecarboxamide

To a cooled solution of the title B compound (9.3 g, 31.6 mmol) in 120 ml of anhydrous DMA was added chloroactylchloride (5.3 g, 46.9 mmol). The solution was stirred at room temperature for 1 hour. The mixture was cooled, 20 ml of saturated aqueous NaHCO₃ solution added and the mixture was concentrated in vacuo. The residue was dissolved in 200 ml of EtOAc and extracted with H₂O (2 x 50 ml) and brine (50 ml). The organic layer was dried over anhydrous MgSO₄ and evaporated to dryness. To deprotect the acetate groups, the residue was dissolved in 130 ml of MeOH. To this solution, a solution of 230 mg Na in 5 ml MeOH was added. It was stirred at room temperature for 1 hour. Dowex 50 (H⁺ form) was added until pH 7. The resin was filtered and the solution was concentrated to 50 ml volume. Crystallization of the product gave 6.2 g of solid title C comound (21.6 mmol), m.p. 184.6-185.5°C. Elemental analysis calc'd for C₁₂H₁₅N₂O₄Cl:
C, 50.40; H, 5.39; N, 9.48; Cl, 12.00;
Found: C, 50.78; H, 5.28; N, 9.59; Cl, 12.19.

### D. 10-[2-[[4-[[(2,3-dihydroxypropyl)amino]-carbonyl]phenyl]amino]-2-oxoethyl]-1,4,7,10-tetraazacyclododecane-1,4,7-triacetic acid

DO3A sulfate (6.0 g, 13.5 mmol) was dissolved in 200 ml of H₂O and the pH of the solution was adjusted to 9.8 by adding 10 N NaOH. To this solution at 85°C was added a solution of the title C compound (5.8 g, 20.2 mmol) in 200 ml of EtOH over a period of 45 minutes. The pH was maintained at 9.8 by adding 5 N NaOH. As the reaction proceeded the mixture turned clear. The mixture was heated at 85°C for 26 hours. The solvents were removed in vacuo. The crude material was dissolved in 500 ml of H2O and applied to a 2-liter column of CHP-20P resin. The column was eluted with a gradient of Et₃NH⁺⁻HCO3 buffer, 5 mM to 100 mM (4 liters each), then 100 mM to 200 mM (1 liter each). The fractions containing the desired compound were combined and concentrated in vacuo. The title D compound (6.1 g) was obtained as mono triethylammonium salt (8.8 mmol).
Elemental analysis calc'd for C₃₂H₅₅N₇O₁₀ · 0.29H₂O:
C, 54.67; H, 7.97; N, 13.95;
Found: C, 54.71; H, 8.14; N, 13.94.

### F. 10-[2-[[4-[[(2,3-Dihydroxypropyl)amino]-carbonyl]phenyl]amino]-2-oxoethyl]-1,4,7,10-tetraazacyclododecane-1,4,7-triacetic acid, monogadolinium salt

700 mg (1.0 mmol) of the title D compound (mono triethylammoniun salt) was dissolved in 10 ml of H₂O and the pH of the solution adjusted to 4.5 by adding diluted HOAc. To this solution was added a solution of Gd(OAc)3·4H₂O (540.4 mg, 1.3 mmol) in 15 ml of H₂O. The mixture was stirred at 45°C for 24 hours. The solution was then diluted to 100 ml and applied to a 600 ml column of CHP-20 resin. The column was eluted with H₂O, then with increasing amount of EtOH (5-20%). Evaporation of the combined fractions containing the desired product afforded 300 mg of the pure title compound (0.40 mmol).
Elemental analysis calc'd for C₂₆H₃₇N₇O₁₀Gd·0.82H₂O:
C, 40.79; H, 5.09; N, 10.98;
Found: C, 40.81; H, 5.14; N, 10.91.

### Example 6

### 10-[N-(4 Nitrophenyl)acetamido]-1,4,7,10-tetraazacyclododecane-1,4,7-triacetic acid, monogadolinum salt

### A. 10-[N-(4 Nitrophenyl)acetamido]-1,4,7,10-tetraazacyclododecane-1,4,7-triacetic acid

A solution of 2-chloro-4'-nitroacetanilide (3 g, 14 mmol) in DMSO (30 ml) was slowly added into a solution of DO3A (5.8 g, 16.8 mmol) in water (30 ml) whose pH was adjusted to 10 by the addition of 10 N NaOH at 50°C. The reaction was maintained at 50°-60°C and the pH was kept at 10 for 54 hours. The yellow precipitate was filtered and dissolved in water (150 ml). The pH of the resulting solution was adjusted to ca.2 by the addition of 1.0 N HCl. The resulting solution was then applied to a 600 ml column of CHP-20P resin. The column was eluted with water (3L), 5% (1L), 10% (1L) and 20% (1.5L) of EtOH in sequence. The fractions containing the desired compound were combined and concentrated in vacuo to give the yellow title A product (2.6 g).
Analysis calc'd for C₂₂H₃₂N₆O₉ 1.30 H₂O:
C, 48.23; H, 6.36; N, 15.34;
Found: C, 47.94; H, 6.48; N, 15.72; H₂O, 4.26.

### B. 10-[N-(4 Nitrophenyl)acetamido]-1,4,7,10-tetraazacyclododecane-1,4,7-triacetic acid, monogadolinum salt

The title A free acid (580 mg, 1.114 mmol) suspended in water (5 mL) was treated with gadolinium acetate (602 mg, 1.48 mmol, 1.33 eq.) in water (3.5 mL) at 65°C. Upon mixing the starting materials the solution became homogeneous but after 25 minutes pale yellow solid was precipitated ou; Filtration and washing of the solid with water (2 mL x 2) gave the title product (470 mg).
Analysis calc'd for C₂₂H₂₉N₆O₉Gd·0.69 H₂O:
C, 38.23; H, 4.43; N, 12.16;
Found: C, 38.34; H, 4.48; N, 12.09; H₂O, 1.80%

### Example 7

### 10-[N-(4-aminophenyl)acetamido]-1,4,7,10-tetraazacyclododecane-1,4,7-triacetic acid, monogadolinium salt

### A. 10-[N-(4-Aminophenyl)acetamido]-1,4,7,10-tetraazacyclododecane-1,4,7-triacetic acid, monotriethylammonium salt

To a solution of compound A of Example 6 (5.3 g, 10.1 mmol) in water (150 ml) whose pH was adjusted to 7.0 by the addition of 10 N NaOH was added 10% Pd/C catalyst (2.17 g, 1.0 mmol of Pd). The solution was hydrogenated at room temperature under hydrogen atmosphere (20-25 psi) for 3 hours. The reaction mixture was then filtered to remove the catalyst. The filtrate was concentrated and applied on a 5 x 20 cm column of DEAE Sephadex resin. The column was eluted with 5 mM, 10 mM, 25 mM, 40 mM, 80 mM and 100 mM of triethylammonium bicarbonate buffer (1 L each). The fractions containing the desired compound was combined and concentrated to yield 4.2 g of the title A mono-triethylammonium salt.

### B. 10-[N-(4-aminophenyl)acetamido]-1,4,7,10-tetraazacyclododecane-1,4,7-triacetic acid, monogadolinium salt

To a solution of the title A compound (3.39 g, 5 mmol) in MeOH (95 ml) and water (18 ml) was added 10% Pd/C catalyst (1.06 g, 0.5 mmol of Pd). The solution was hydrogenated at room temperature under hydrogen atmosphere (20-25 psi) for 10 hours. The solution containing the catalyst was then filtered. After the filtrate was evaporated to dryness, the residue was crystallized from MeOH (30 ml) to give the product (3.04 g).
Analysis calc'd for C₂₂H₃₃N₇O₇Gd·4.18 H₂O:
C, 36.49; H, 5.48; N, 11.61;
Found: C, 36.22; H, 5.41; N, 11.41; H₂O, 10.4%.

### Examples 8 and 9

### 10-[[N-(4-(N'-Isothiocyanato)phenyl]acetamido]]-1,4,7,10-tetraazacyclododecane-1,4,7-triacetic acid, monogadolinium salt

and

### 10-[N-[4-(N'-Methylthioureido)phenyl]acetamido]-1,4,7,10-tetraazacyclododecane-1,4,7-triacetic acid, monogadolinium salt

To a solution of the title compound from Example 7 (194.7 mg, 0.3 mmol) in H₂O (7.5 ml) was added a solution of thiophosgene (138 mg, 1.2 mmol) in CHCl₃ (6 ml). the biphasic mixture was stirred at room temperature until the compound was consumed completely. The aqueous layer (pH 1.0-1.5) was separated, and the CHCl₃ layer was washed with water (1 ml x 2). The combined aqueous layers were treated with 1 N NaOH to adjust the pH of the so-formed title 8 solution to 6,0. Methylamine (18.04 mg, 0.58 mmol) was then added, and the reaction mixture was stirred for 10 minutes. The resulting solution was loaded on a column and eluted with water and ethanol. The desired title 9 compound was eluted out by 10% of ethanol to give the desired product (129 mg).
Analysis calc'd for C₂₄H₃₄N₇O₇SGd·2.99 H₂O:
C, 37.16; H, 5.19; N, 12.64;
Found: C, 37.00; H, 5.16; N, 12.39; H₂O, 6.94%.

### Example 10

### 10-[N-[4-(N',N'-Diethylaminothioureido)phenyl]-acetamido]-1,4,7,10-tetraazacyclododecane-1,4,7-triacetic acid, monogadolinium salt

To a solution of the title gadolinium chelate of Example 7 (324 mg, 0.5 mmol) in H₂O (15 ml) was added a solution of thiophosgene (230 mg, 2 mmol) in CHCl₃ (10 ml). The biphasic mixture was stirred at room temperature until the chelate was consumed completely to provide a solution of the Example 8 isothiocyanato product. The aqueous layer (pH 1.0-1.5) was separated, and the CHCl₃ layer was washed with water (2 ml x 2). The combined aqueous layers were treated with 1 N NaOH to adjust the pH of the isothiocyanto solution to 6.0. Diethylamine (73.1 mg, 1.0 mmol) was then added, and the reaction mixture was stirred for 10 minutes. The resulting solution was loaded on CHP 20P column and eluted with water and ethanol. The desired compound was eluted out by 10% ethanol to give the desired product (286 mg).
Analysis calc'd for C₂₇H₄₀N₇O₇SGd 2.31 H₂O:
C, 40.26; H, 5.58; N, 12.17;
Found: C, 40.30; H, 5.71; N, 11.99; H₂O, 5.16%.

### Example 11

### 10,10'[[[[[(1,2-Ethanediyl)diimino]bis(thioxomethyl)-diimino]-bis(4,1-phenylene)]diimino-bis(2-oxo-2,1-ethanediyl)]bis[1,4,7,10-tetraazacyclododecane-1,4,7-triacetic acid], gadolinium (1:2) salt

The Example 8 isothiocyanato derivative solution was prepared as in Examples 8, 9 and 10. Ethylenediamine (11.2 mg, 0.19 mmol) dissolved in water (1.0 mL) was added to this solution. The pH of the resulting mixture was initially increased to 10.04 and then decreased to 7.88 at the end of 3 hours stirring. Concentrated ammonium hydroxide was used to clean up the excess Example 8 chelate. The crude product, obtained after removal of the water and ammonium hydroxide, was purified by CHP 20P (75-150 µ) chromatography (2.5 x 20 cm). The desired product was eluted out by 10% ethanol to give the dimeric gadolinium chelate (150 mG).
Analysis calc'd for C₄₈H₆₆N₁₄O₁₄S₂Gd₂·2.19 H₂O:
c, 38.92; H, 4.79; N, 13.24; S. 4.33;
Found: C, 39.07; H, 4.77; N, 13.19; S, 3.95; H₂O, 2.66%

### Example 12

### N,N'-bis[N"-[2-(1,4,7,10-Tetraazacyclododecane-1,4,7-triacetic acid)-1-oxoethyl]aminophenyl]thiourea, gadolinium (1:2) salt

The product of Example 7 (194.7 mg, 0.3 mmol) dissolved in water (0.5 ml) was added to the Example 8 isothocyanato solution described above. The reaction mixture was stirred at room temperature for 10 hours. The resulting solution was applied to a CHP 20P column. The column was eluted with H₂O, 2%, 4% and 6% of EtOH in sequence. The desired compound was eluted by 6% of EtOH to give the title product (259 mg).
Analysis calc'd for C₄₅H₆₀N₁₂O₁₄SGd₂·4.37H₂O:
C, 38.11; H, 4.88; N, 11.85;
Found: C, 38.35; H, 5.03; N, 11.80; H₂O, 5.55%.

### Example 13

### 10,10'-[[[[[[Iminotris(1,2-ethanediyl)triimino]-tris(thioxomethyl)]-triimino]tris-(4,1-phenylene)]-triimino-tris(2-oxo-2,1-ethanediyl)]tris[1,4,7,10-tetraazacyclo-dodecane-1,4,7-triacetic acid], gadolinium (1:3) salt

Tris(2-aminoethyl)amine (19.7 mg, 0.135 mmol) dissolved in water (0.5 mL) was added to the Example 8 isothiocyanato solution described above. The pH of the resulting mixture read 10.10 upon mixing, and then decreased to 7.88 after stirring 18 hours. Concentrated ammonium hydroxide was added to quench the excess Example 8 compound. The crude product, which was obtained after removal of the water and ammonium hydroxide, was purified by CHP 20P (75-150 µ) chromatography (2.5 x 20 cm). The desired product was eluted by 20% ethanol to give the trimeric gadolinium chelate (230 mG).
Analysis calc'd for C₇₅H₁₀₅N₂₂O₂₁S₃Gd₃·6.92H₂O:
C, 38.44; H, 5.11; N, 13.15; S, 4.10
Found: C, 38.75; H, 5.09; N, 13.14; S, 3.76; H₂O, 5.32%

### Example 14

### 10-[2-[[2-(4-Nitrophenyl)ethyl]amino]-2-oxoethyl]-1,4,7,10-tetraazacyclododecane-1,4,7-triacetic acid, monogadolinium salt

### A. 2-Chloro-N-[2-(4-nitrophenyl)ethyl]acetamide

To a solution of p-nitrophenethylamine (hydrochloride salt, 6.0 g, 29.7 mmol) in anhydrous DMA (50 ml) and Et₃N (3.0 g, 29.7 mmol) was added chloroacetyl chloride (6.71 g, 59.4 mmol). The reaction mixture was stirred at room temperature for 2 hours. The solvent was removed in vacuo, the residue was dissolved in EtOAc and the solution was washed with aqueous NaHCO₃ (30 ml) and brine (30 ml). The organic layer was dried and the solvent removed to obtain the crude product as a yellow solid. Recrystallization of this material from hot EtOAc/ hexanes (10:1) afforded the anilide as a white crystal (5.5 g).
Elemental analysis calc'd C₁₀H₁₁N₂ClO₃:
C, 49.50; H, 4.57; N, 11.54; Cl, 14.61; O, 19.78;
Found: C, 49.74; H, 4.50; N, 11.12; Cl, 14.35.

### B. 10-[2-[[2-(4-Nitrophenyl)ethyl]amino]-2-oxoethyl-1,4,7,10-tetraazacyclododecane-1,4,7-triacetic acid

DO3A sulfate (6.0 g, 13.5 mmol) was dissolved in H₂O (100 ml) and the pH of the solution was adjusted to 9.5 by adding 10 N NaOH. To this solution at 80°C was added a solution of the title A compound (5.5 g, 22.7 mmol) in EtOH (80 ml) over a period of 30 minutes. The pH was maintained at 9.5 by adding 5 N NaOH. The mixture was heated at 80°C for 48 hours, and the solvents were removed in vacuo. The solid was dissolved in H₂O (200 ml) and washed with EtOAc (2 x 50 ml). The aqueous layer was evaporated by a water pump at 40°C to remove trace of EtOAc. The solution was diluted to 600 ml and applied to a 1.5-liter column of DEAE sephadex resin. The column was eluted with a gradient of Et₃NH⁺⁻HCO₃ buffer, 5 mM to 100 mM (4 liter each), then 100 mM to 200 mM (1.5 liter each). The fractions containing the desired compound were combined and concentrated in vacuo. The title B compound (7.67 g) was obtained as mono triethylammonium salt.

### C. 10-[2-[[2-(4-Nitrophenyl)ethyl]amino]-2-oxoethyl]-1,4,7,10-tetraazacyclododecane-1,4,7-triacetic acid, monogadolinium salt

The title B compound (mono triethylammonium salt, 65.3 mg, 0.1 mmol) was dissolved in H₂O (5 ml) and the pH of the solution adjusted to 4.5 by adding diluted HOAc. To this solution was added a solution of Gd(OAc)₃·4H₂O (52.8 mg, 0.13 mmol) in H₂O (5 ml). The mixture was stirred at room temperature for 1 hour. The solution was then applied to a 400 ml column of CHP-20 resin, using EtOH/H₂O (0-15%) as an eluent. The fractions containing the desired compound were combined and removal of the solvent afforded the title compound as a monogadolinium salt (450 mg).
Elemental analysis calc'd for C₂₄H₃₃N₆GdO₉·1.93 H₂O:
C, 38.88; H, 5.01; N, 11.33; Gd, 21.21; O, 23.57;
Found: C, 38.81; H, 5.15; N, 11.40.

## Claims (Claims for the following Contracting State(s): AT, BE, CH, DE, DK, FR, GB, GR, IE, IT, LI, LU, MC, NL, PT, SE)

1. A diagnostic agent comprising an aminocarboxylate ligand, complexed with a paramagnetic metal ion, of formula Ia 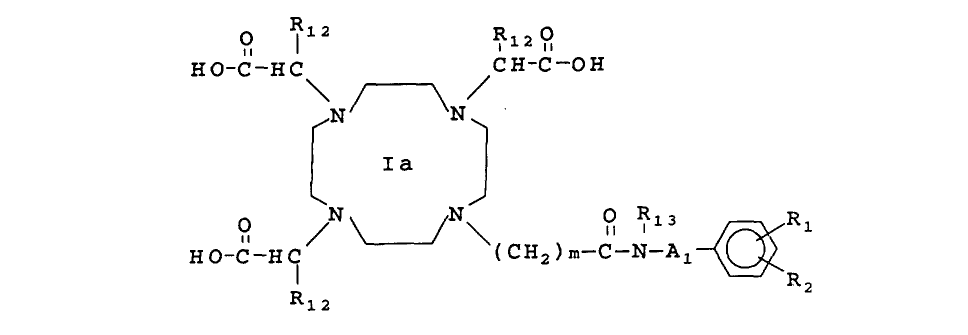 in which A₁ is -(CH₂)_{m'}- or a bond;
-(CH₂)ₘ- and -(CH₂)_{m'}- may independently be substituted with alkyl or hydroxyalkyl;
R₁ and R₂ are independently H, alkyl, -NO₂, -NH₂, 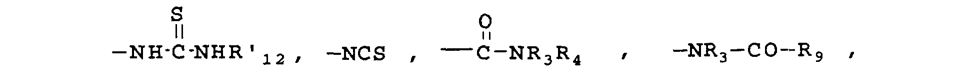 where R₉ is alkyl or hydroxyalkyl, with the proviso that at least one of R₁ and R₂ is not hydrogen;
R₃ and R₄ are independently H, alkyl, aralkyl, aryl, alkoxy, and hydroxyalkyl;
R₁₂ is H, alkyl or hydroxyalkyl;
R'₁₂ is H alkyl or hydroxyalkyl; R₁₃ is H, alkyl, aralkyl,aryl, alkoxy, hydroxyalkyl;
m and m' are independently 1 to 5; and multimeric forms thereof.

2. A diagnostic agent of claims 1, in which the paramagnetic metal is Gd.

3. A compound of multimeric forms of that of formula Ia as defined in claim 1.

4. A compound of formula Ia, wherein R₁ and R₂ are -CO-NR₃R₄, and R₃ is hydroxyalkyl.

5. A compound of formula Ia, in which R₁ and R₂ are -CO-NHR₃ wherein R₃ is selected from -CH₂-CHOH-CH₂OH and -CH(CH₂OH)2.

6. A compound of formula Ia, in which R₁ and R₂ are -CONH-CH₂-CHOH-CH₂OH or -CONH-CH(CH₂OH)₂.

7. Compounds of formula Ia i.e.
10-[2-[[3,5-bis[[(2,3-dihydroxypropyl)amino]carbonyl]phenyl]amino]-2-oxoethyl]-1,4,7,10-tetraazacyclododecane-1,4,7-triacetic acid;
10-[2-[[3,5-bis-[[[2-hydroxy-1-(hydroxymethyl)ethyl]amino]carbonyl]phenylamino]2-oxoethyl]-1,4, 7,10-tetraazacyclododecane-1,4,7-triacetic acid
10-[2-[methyl[3,5-bis[[(2-methylbutyl)amino]carbonyl]phenyl]amino]-2-oxoethyl]-1,4,7,10-tetraazacyclododecane-1,4,7-triacetic acid;
10-[2-[[4-[[2,3-dihydroxypropyl)amino]carbonyl]phenyl]amino]-2-oxoethyl-1,4,7,10-tetraazacyclododecane-1,4,7-triacetic acid;
10-[N-(4 nitrophenyl)acetamido]-1,4,7,10-tetraazacyclododecane-1,4,7-triacetic acid;
10-[N-(4-aminophenyl)acetamido]-1,4,7,10-tetraazacyclododecane-1,4,7-triacetic acid;
10-[[N-{4-(N'-isothiocyanto)phenyl]acetamido]]-1,4,7,10-tetraazacyclododecane-1,4,7-triacetic acid;
10-[N-[4-(N'-methylthioureido)phenyl]acetamido]-1,4,7,10-tetraazacyclododecane-1,4,7-triacetic acid;
10-[N-[4-(N',N'-diethylaminothioureido)phenyl]acetamido]-1,4,7,10-tetraazacyclododecane-1,4, 7-triacetic acid ;
10,10'[[[[[(1,2-ethanediyl)diimino]bis(thioxomethyl)diimino]-bis(4,1-phenylene)]diimino-bis(2-oxo-2, 1-ethanediyl)]bis[1,4,7,10-tetraazacyclododecane-1,4,7-triacetic acid];
N,N'-bis[N"-[2-(1,4,7,10-tetraazacyclododecane-1,4,7-triacetic acid)-1-oxoethyl]aminophenyl]thiourea;
10,10'-[[[[[[iminotris(1,2-ethanediyl)triimino]tris(thioxomethyl)]-triimino]tris-(4,1-phenylene)]triimino-tris(2-oxo-2,1-ethanediyl)]tris[1,4,7,10-tetraazacyclo-dodecane-1,4,7-triacetic acid]; or
10[2-[[2-(4-nitrophenyl)ethyl]amino]-2-oxoethyl]-1,4,7,10-tetraazacyclododecane-1,4,7-triacetic acid.

8. The Gd complexes of the compounds of claim 7.

9. A complex of a metal atom and a metal chelating ligand of compounds according to claims 4-8, or a pharmaceutically acceptable salt of such complex.

10. The complex of claim 9, in which the said -metal atom has atomic number 56-83, e.g. Gd.

11. Multimer compounds selected from formulae 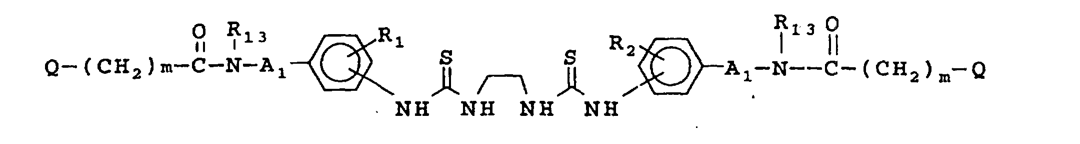 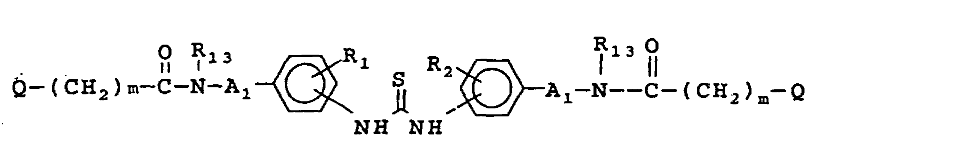 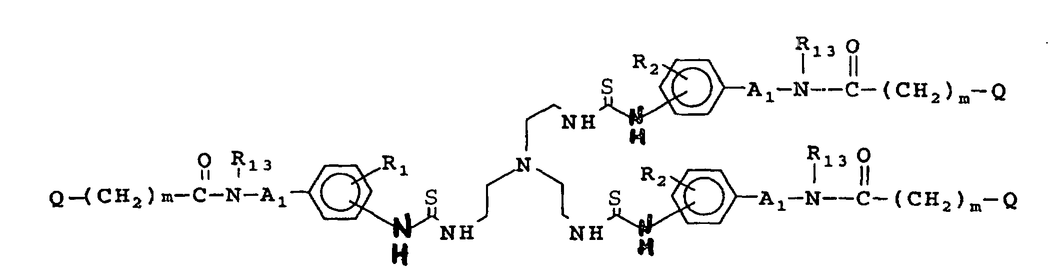 or 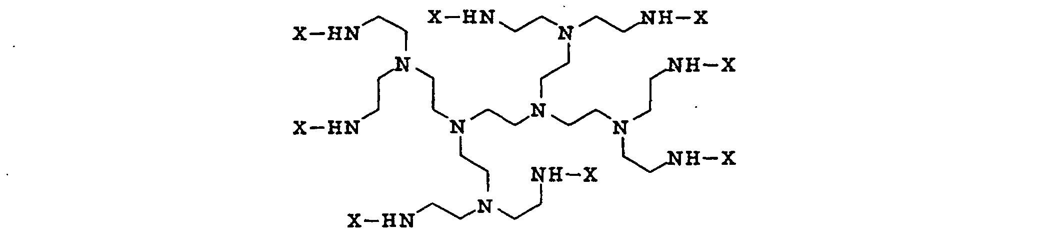 where X is a group of formula 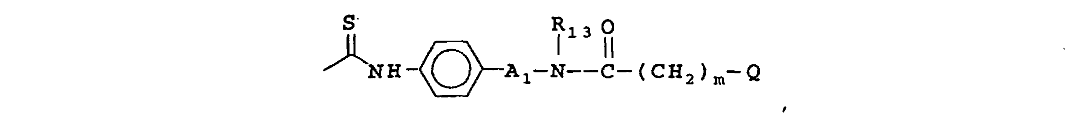 and Q is an amino carboxylate ligand defined in claim 1, the other variables being defined as in claim 1.

## Claims (Claims for the following Contracting State(s): ES)

1. A process for preparing a compound of formula Ia 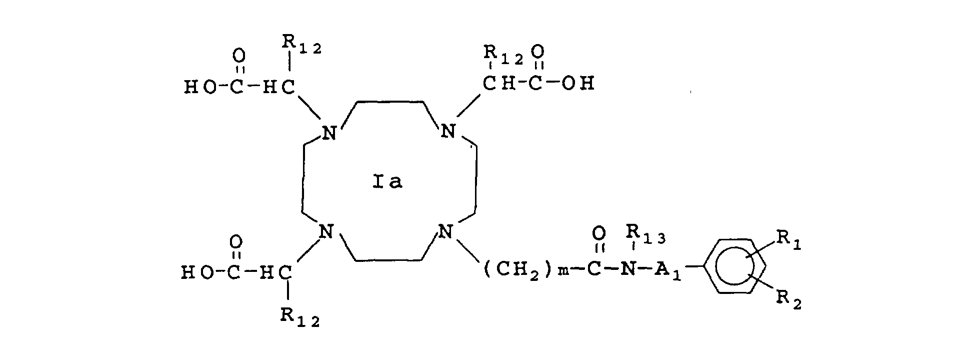 in which A₁ is -(CH₂)_{m'}- or a bond;
-(CH₂)ₘ- and -(CH₂)_{m'}- may independently be substituted with alkyl or hydroxyalkyl;
R₁ and R₂ are independently H, alkyl, -NO₂, -NH₂, 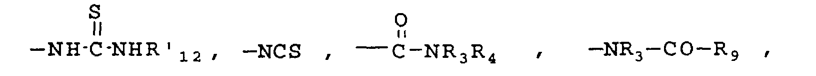 where R₉ is alkyl or hydroxyalkyl, with the proviso
that at least one of R₁ and R₂ is not hydrogen;
R₃ and R₄ are independently H, alkyl, aralkyl, aryl, alkoxy, and hydroxyalkyl; R₁₂ is H, alkyl or hydroxyalkyl;
R'₁₂ is H alkyl or hydroxyalkyl; R₁₃ is H, alkyl, aralkyl, aryl, alkoxy, hydroxyalkyl; m and m' are independently 1 to 5; and multimeric forms thereof.
which process comprises reacting a compound of the formula 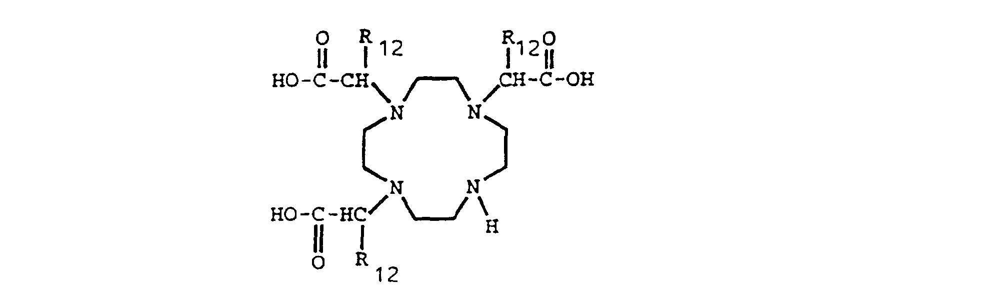 with a compound of the formula 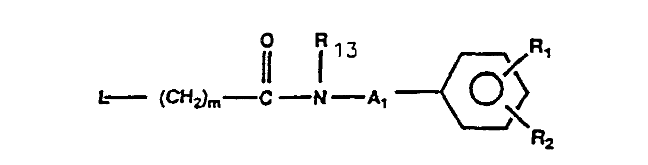 (where L is a leaving group, e.g. halogen) in a solvent and in the presence of a base.

2. A process of claim 1 wherein R₁ and R₂ are each 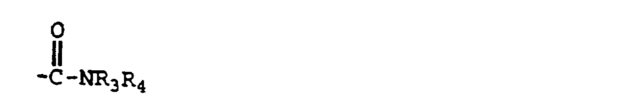 wherein each R₃ group is hydroxyalkyl.

3. A process of claim 1 wherein R₁ and R₂ are each 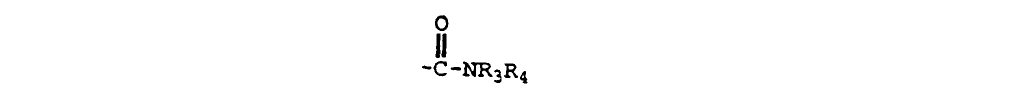 wherein each R₃ group is selected from 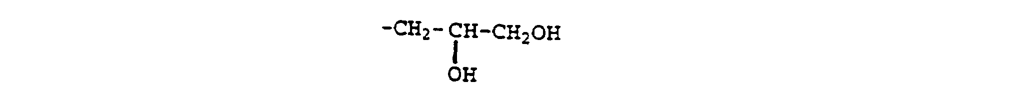 and -CH(CH₂OH)₂, and wherein each R₄ group is hydrogen.

4. A process of claim 1 wherein R₁ and R₂ are each

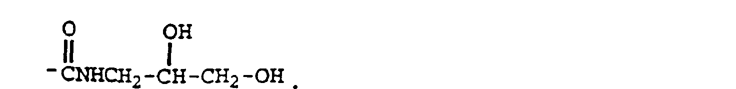

5. A process of claim 1 wherein R₁ and R₂ are each

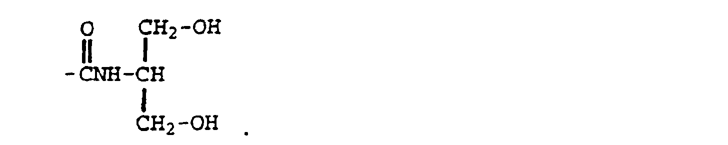

6. A process of claim 1 wherein the product is 10-[2-[[3,5-bis[[(2,3-dihydroxypropyl)amino]carbonyl]phenyl]amino]-2-oxoethyl]-1,4,7,10-tetraazacyclododecane-1,4,7-triacetic acid;
10-[2-[[3,5-bis-[[[2-hydroxy-1-(hydroxymethyl)ethyl]amino]carbonyl]phenylamino]2-oxoethyl]-1,4, 7,10-tetraazacyclododecane-1,4,7-triacetic acid
10-[2-[methyl[3,5-bis[[(2-methylbutyl)amino]carbonyl]phenyl]amino]-2-oxoethyl]-1,4,7,10-tetraazacyclododecane-1,47-triacetic acid;
10-[2-[[4-[[2,3-dihydroxypropyl)amino]carbonyl]phenyl]amino]-2-oxoet hyl-1,4,7,10-tetraazacyclododecane-1,4,7-triacetic acid;
10-[N-(4 nitrophenyl)acetamido]-1,4,7,10-tetraazacyclododecane-1,4,7-triacetic acid;
10-[N-(4-aminophenyl)acetamido)1,4,7,10-tetraazacyclododecane-1,4,7-triacetic acid:
10-[[N-{4-(N'-isothiocyanto)phenyl]acetamido]]-1,4,7,10-tetraazacydododecane-1,4,7-triacetic acid;
10-[N-[4-(N'-methylthioureido)phenyl]acetamido]-1,4,7,10-tetraazacyclododecane-1,4,7-triacetic acid;
10-[N-[4-(N',N'-diethylaminothioureido)phenyl]acetamido]-1,4,7,10-tetraazacyctododecane-1,4, 7-triacetic acid ;
10,10'[[[[[(1,2-ethanediyl)diimino]bis(thioxomethyl)diimino]-bis(4,1-phenylene)]diimino-bis(2-oxo-2, 1-ethanediyl)]bis[1,4,7,10-tetraazacyclododecane-1,4,7-triacetic acid];
N,N'-bis[N"-[2-(1,4,7,10-tetraazacydododecane-1,4,7-triacetic acid)-1-oxoethyl]aminophenyl]thiourea;
10,10'-[[[[[[iminotris(1,2-etanediyl)triimino]tris(thioxomethyl)]-triimino]tris-(4,1-phenylene)]triimino-tris(2-oxo-2,1-ethanediyl)]tris[1,4,7,10-tetraazacyclo-dodecane-1,4,7-triacetic acid]; or
10-[2-[[2-(4-nitrophenyl)ethyl]amino]-2-oxoethyl-1,4,7,10-tetraazacydododecane-1,4,7-triacetic acid.

7. A process of claim 1 or 2 wherein the paramagnetic metal ion is gadolinium and the ligand is a compound named in claim 6.

8. A process for preparing a complex of an aminocarboxylate ligand and a metal ion, or a pharmaceutically acceptable salt of such a complex, wherein the aminocarboxylate ligand is a compound of formula la as defined in any one of claims 1-5, which process comprises reacting the aminocarboxylate ligand with a source of the metal ion.

9. A process of claim 8 wherein said metal atom is of atomic number 56-83.

10. A process of claim 8 wherein said metal is gadolinium (III).

11. A process of claim 1 wherein the product is a multimer selected from 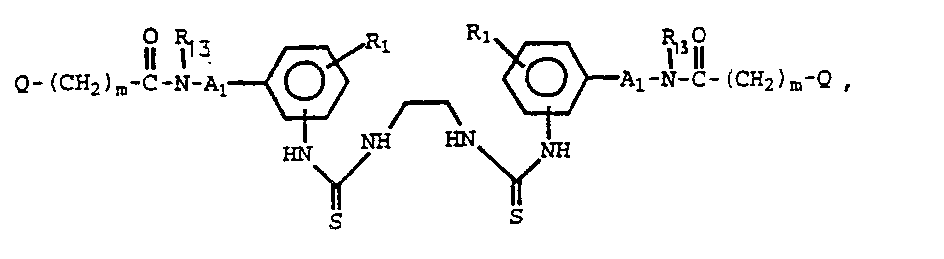 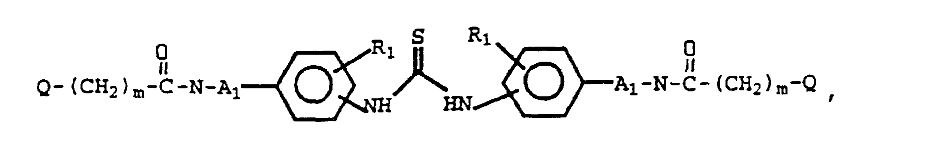 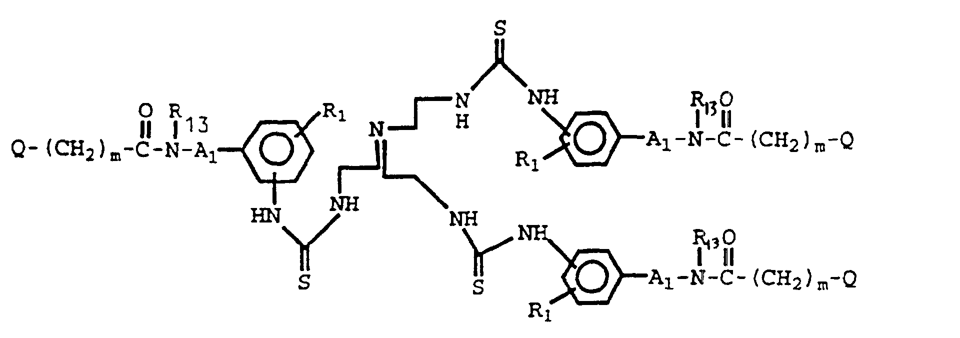 or 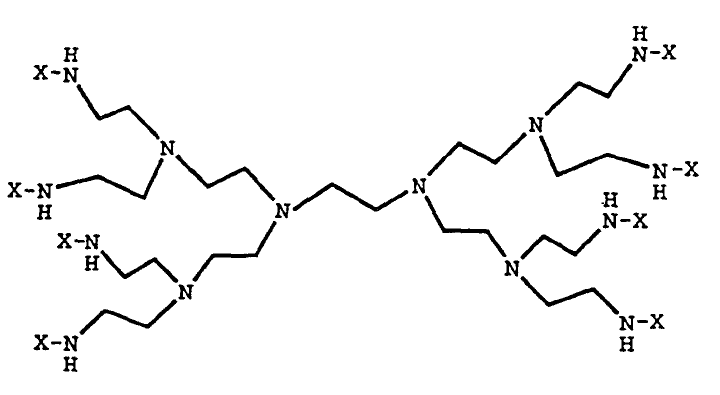 where X = 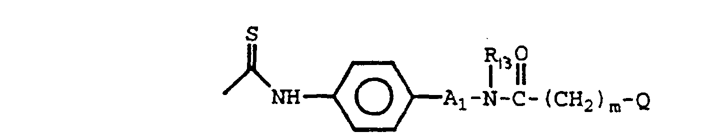 and wherein Q is an aminocarboxylate ligand and the other variables are as defined in claim 1.

## Patentansprüche (Patentansprüche für folgende(n) Vertragsstaat(en): AT, BE, CH, DE, DK, FR, GB, GR, IE, IT, LI, LU, MC, NL, PT, SE)

1. Diagnostisches Mittel enthaltend einen mit paramagnetischen Metallionen komplexierten Aminocarboxylat Liganden gemäß der Formel Ia 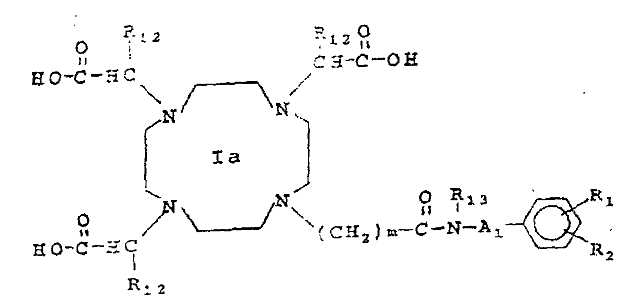 in der A₁ -(C*H*₂)_{m'}- oder eine Bindung ist;
-(CH₂)ₘ- und -(CH₂)_{m'}- unabhängig voneinander mit Alkyl oder Hydroxyalkyl substituiert sein können;
R₁ und R₂ unabhängig voneinander H, Alkyl, -NO₂, -NH₂, 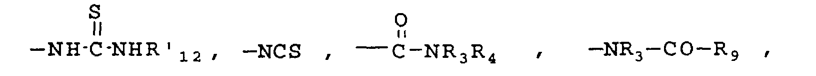 sind, wobei R₉ Alkyl oder Hydroxyalkyl ist, unter der Bedingung, daß mindestens einer der Reste R₁ und R₂ nicht Wasserstoff ist;
R₃ und R₄ unabhängig voneinander H, Alkyl, Aralkyl, Aryl, Alkoxy und Hydroxyalkyl sind;
R₁₂ H, Alkyl oder Hydroxyalkyl ist;
R'₁₂ H, Alkyl oder Hydroxyalkyl ist;
R₁₃ H, Alkyl, Aralkyl, Aryl, Alkoxy, Hydroxyalkyl ist;
m und m' unabhängig voneinander 1 bis 5 sind; und multimere Formen davon.

2. Diagnostisches Mittel nach Anspruch 1, in dem das paramagnetische Metall Gd ist.

3. Verbindung von multimeren Formen von der von Formel Ia wie in Anspruch 1 definiert.

4. Verbindung der Formel Ia, in der R₁ und R₂ -CO-NR₃R₄ sind und R₃ Hydroxyalkyl ist.

5. Verbindung der Formel Ia, in der R₁ und R₂ -CO-NHR₃ sind, wobei R₃ aus -CH₂-CHOH-CH₂OH und -CH(CH₂OH)₂ ausgewahlt ist.

6. Verbindung der Formel Ia, in der R₁ und R₂ -CONH-CH₂-CHOH-CH₂OH oder -CONH-CH(CH₂OH)₂ sind.

7. Vebindungen der formel Ia, d.h.
10-[2-[[3,5-Bis[[(2,3-dihydroxypropyl)amino]carbonyl]-phenyl]amino]-2-oxoethyl]-1,4,7,10-tetraazacyclododecan-1,4,7-triessigsäure;
10-[2-[[3,5-Bis[[[2-hydroxy-1-(hydroxymethyl)ethyl]amino]carbonyl]phenylamino]-2-oxoethyl]-1,4,7,10-tetraazacyclododecan-1,4,7-triessigsäure;
10-[2-[Methyl[3,5-bis[[(2-methylbutyl)amino]carbonyl]-phenyl]amino]-2-oxoethyl]-1,4,7,10-tetraazacyclododecan-1,4,7-triessigsäure;
10-[2-[[4-[[2,3-Dihydroxypropyl)amino]carbonyl]phenyl]-amino]-2-oxoethyl-1,4,7,10-tetraazacyclododecan-1,4,7-triessigsäure;
10-[N-(4-Nitrophenyl)acetamido]-1,4,7,10-tetraazacyclododecan-1,4,7-triessigsäure;
10-[N-(4-Aminophenyl)acetamido]-1,4,7,10-tetraazacyclododecan-1,4,7-triessigsäure;
10-[[N-{4-(N'-Isothiocyanto)phenyl]acetamido]]-1,4,7,10-tetraazacyclododecan-1,4,7-triessigsäure;
10-[N-[4-(N'-Methylthioureido)phenyl]acetamido]-1,4,7,10-Tetraazacyclododecan-1,4,7-triessigsäure;
10-[N-[4-(N',N'-Diethylaminothioureido)phenyl]acetamido]-1,4,7,10-tetraazacyclododecan-1,4,7-triessigsäure;
10,10'[[[[[(1,2-Ethandiyl)diimino]bis(thioxomethyl)diimino]-bis(4,1-phenylen)]diimino-bis(2-oxo-2,1-ethandiyl)]bis[1,4,7,10-tetraazacyclododecan-1,4,7-triessigsäure];
N,N'-Bis[N"-[2-(1,4,7,10-tetraazacyclododecan-1,4,7-triessigsäure)-1-oxoethyl]aminophenyl]thioharnstoff;
10,10'-[[[[[[Iminotris(1,2-ethandiyl)triimino]tris (thioxomethyl)]-triimino]tris-(4,1-phenylen)]triiminotris(2-oxo-2,1-ethandiyl)]tris[1,4,7,10-tetraazacyclododecan-1,4,7-triessigsäure]; oder
10[2-[[2-(4-Nitrophenyl)ethyl]amino]-2-oxoethyl]-1,4,7,10-tetraazacyclododecan-1,4,7-triessigsäure.

8. Die Gd-Komplexe der Verbindungen gemäß Anspruch 7.

9. Komplex eines Metallatoins und eines Metall chelatisierenden Liganden von Verbindungen gemäß den Ansprüchen 4 bis 8 oder ein pharmazeutisch verträgliches Salz eines solchen Komplexes.

10. Komplex nach Anspruch 9, in dem das Metallatom eine Ordnungszahl von 56 bis 83 hat, z.B. Gd.

11. Multimere Verbindungen ausgewählt unter den Formeln 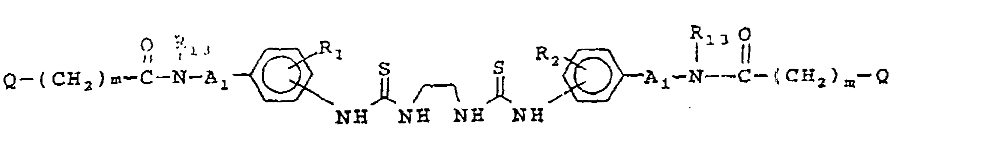 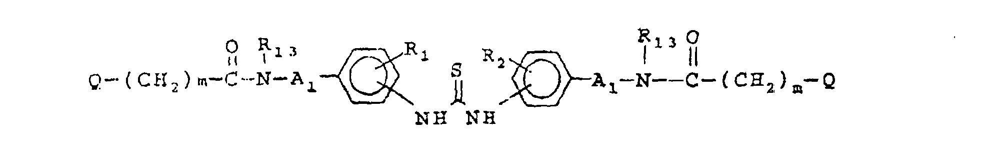 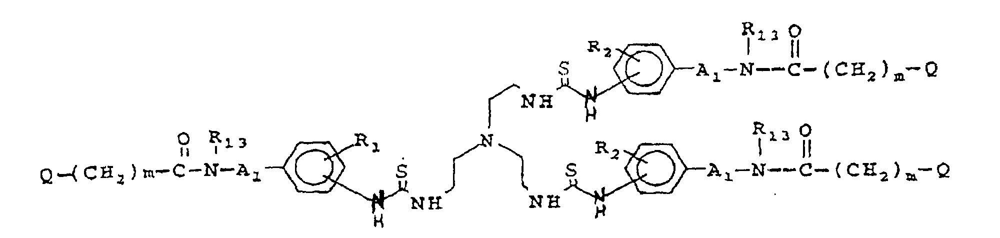 oder 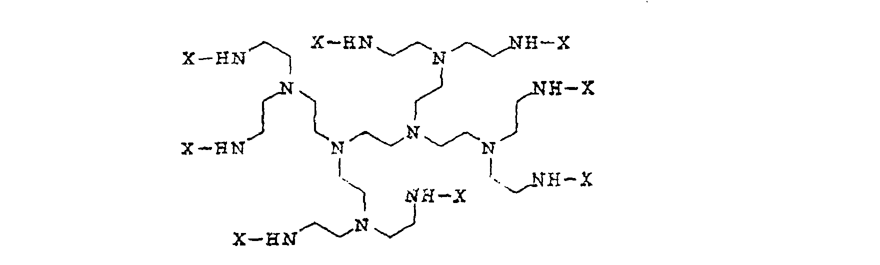 wobei X eine Gruppe der Formel 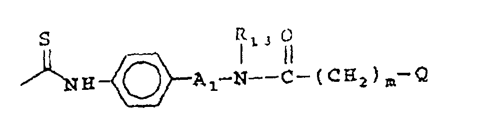 ist und Q ein in Anspruch 1 definierter Aminocarboxylat Ligand ist und die anderen Variablen wie in Anspruch 1 definiert sind.

## Patentansprüche (Patentansprüche für folgende(n) Vertragsstaat(en): ES)

1. Verfahren zur Herstellung einer Verbindung gemäß Formel Ia 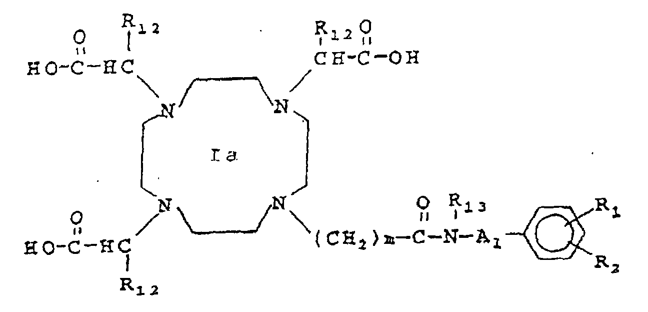 in der A₁-(CH₂)_{m'}- oder eine Bindung ist;
-(CH₂)ₘ- und -(CH₂)_{m'}- unabhängig voneinander mit Alkyl oder Hydroxyalkyl substituiert sein können;
R₁ und R₂ unabhängig voneinander H, Alkyl, -NO₂, -NH₂, 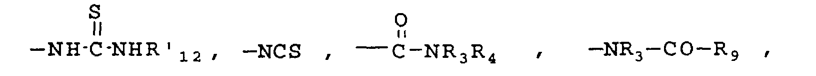 sind, wobei R₉ Alkyl oder Hydroxyalkyl ist, unter der Bedingung, daß mindestens einer der Reste von R₁ und R₂ nicht Wasserstoff ist;
R₃ und R₄ unabhängig voneinander H, Alkyl, Aralkyl, Aryl, Alkoxy und Hydroxyalkyl sind;
R₁₂ H, Alkyl oder Hydroxyalkyl ist;
R'₁₂ H, Alkyl oder Hydroxyalkyl ist;
R₁₃ H, Alkyl, Aralkyl, Aryl, Alkoxy, Hydroxyalkyl ist;
m und m' unabhängig voneinander 1 bis 5 sind; und multimere Formen davon,
wobei das Verfahren das Reagieren einer Verbindung mit der Formel 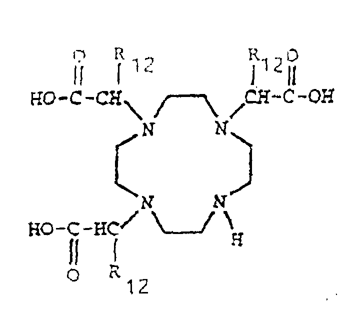 mit einer Verbindung mit der Formel 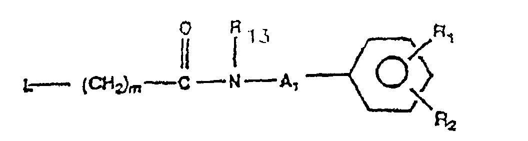 (wobei L eine Abgangsgruppe ist, z.B. Halogen) in einem Lösungsmittel und in Anwesenheit einer Base umfaßt.

2. Verfahren nach Anspruch 1, in dem R₁ und R₂ jeweils 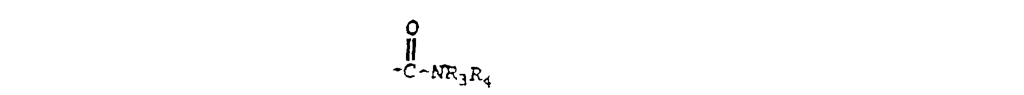 sind und wobei jede R₃-Gruppe Hydroxyalkyl ist.

3. Verfahren nach Anspruch 1, in dem R₁ und R₂ jeweils 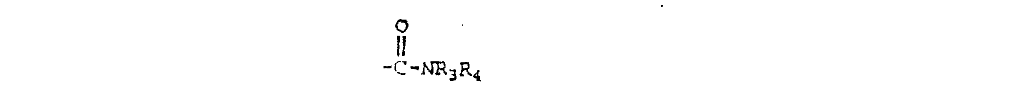 sind, wobei jede R₃ Gruppe unter 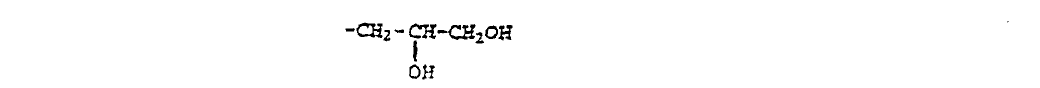 und -CH(CH₂OH₂)₂ ausgewählt ist und wobei jede R₄ Gruppe Wasserstoff ist.

4. Verfahren nach Anspruch 1, in dem R₁ und R₂ jeweils 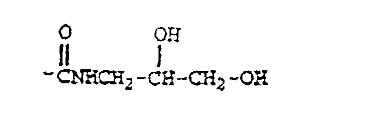 sind.

5. Verfahren nach Anspruch 1, in dem R₁ und R₂ jeweils 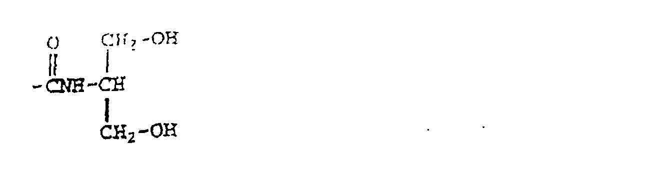 sind.

6. Verfahren nach Anspruch 1, bei dem das Produkt
10-[2-[[3,5-Bis[[(2,3-dihydroxypropyl)amino]carbonyl]-phenyl]amino]-2-oxoethyl]-1,4,7,10-tetraazacyclododecan-1,4,7-triessigsäure;
10-[2-[[3,5-Bis[[[2-hydroxy-1-(hydroxymethyl)ethyl]amino]carbonyl]phenylamino]-2-oxoethyl]-1,4,7,10-tetraazacyclododecan-1,4,7-triessigsäure;
10-[2-[Methyl[3,5-bis[[(2-methylbutyl)amino]carbonyl]-phenyl]amino]-2-oxoethyl]-1,4,7,10-tetraazacyclododecan-1,4,7-triessigsäure;
10-[2-[[4-[[2,3-Dihydroxypropyl)amino]carbonyl]phenyl]-amino]-2-oxoethyl-1,4,7,10-tetraazacyclododecan-1,4,7-triessigsäure;
10-[N-(4-Nitrophenyl)acetamido]-1,4,7,10-tetraazacyclododecan-1,4,7-triessigsäure;
10-[N-(4-Aminophenyl)acetamido]-1,4,7,10-tetraazacyclododecan-1,4,7-triessigsäure;
10-[[N-{4-(N'-Isothiocyanto)phenyl}acetamido]]-1,4,7,10-tetraazacyclododecan-1,4,7-triessigsäure;
10-[N-[4-(N'-Methylthioureido)phenyl]acetamido]-1,4,7,10-Tetraazacyclododecan-1,4,7-triessigsäure;
10-[N-[4-(N',N'-Diethylaminothioureido)phenyl]acetamido]-1,4,7,10-tetraazacyclododecan-1,4,7-triessigsäure;
10,10'[[[[[(1,2-Ethandiyl)diimino]bis(thioxomethyl)diimino] bis(4,1-phenylen)]diimino-bis(2-oxo-2,1-ethandiyl)]bis[1,4,7,10-tetraazacyclododecan-1,4,7-triessigsäure];
N,N'-Bis[N"-[2-(1,4,7,10-tetraazacyclododecan-1,4,7-triessigsäure)-1-oxoethyl)aminophenyl]thioharnstoff;
10,10'-[[[[[[Iminotris(1,2-ethandiyl)triimino)tris (thiozomethyl)]-triimino]tris-(4,1-phenylen)]triiminotris(2-oxo-2,1-ethandiyl)]tris[1,4,7,10-tetraazacyclododecan-1,4,7-triessigsäure]; oder
10[2-[[2-(4-Nitrophenyl)ethyl]amino]-2-oxoethyl]-1,4,7,10-tetraazacyclododecan-1,4,7-triessigsäure.

7. Verfahren nach Anspruch 1 oder 2, in dem das paramagnetische Metallion Gadolinium und der Ligand eine der in Anspruch 6 genannten Verbindungen ist.

8. Verfahren zur Herstellung eines Komplexes eines Aminocarboxilate-Liganden und eines Mettalions oder eines pharmazeutisch verträglichen Salzes eines solchen Komplexes, in dem der Aminocarboxylat-Ligand eine Verbindung der Formel Ia ist, wie in einem der Ansprüche 1 bis 5 definiert, wobei das Verfahren das Reagieren des Aminocarboxylat-Liganden mit einer Quelle für das Metallion umfaßt.

9. Verfahren nach Anspruch 8, in dem das Metallatom eine Ordnungszahl von 56 bis 83 aufweist.

10. Verfahren nach Anspruch 8, in dem das Metall Gadolinium (III) ist.

11. Verfahren nach Anspruch 1, in dem das Produkt ein Multimer ist, das aus 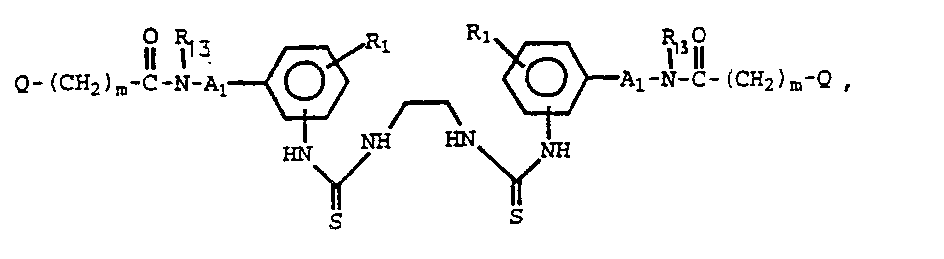 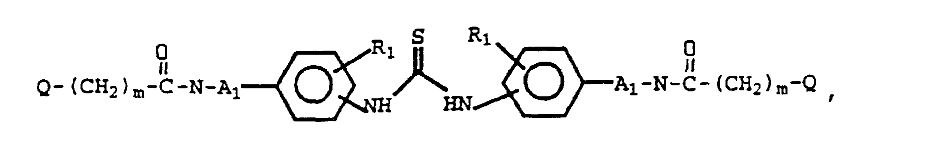 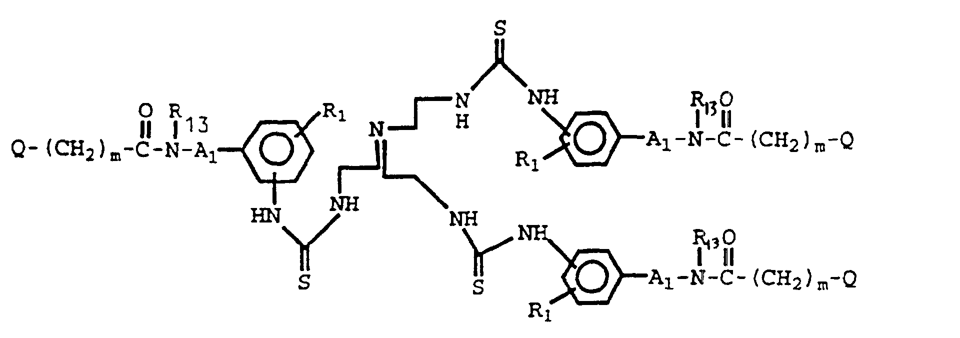 oder 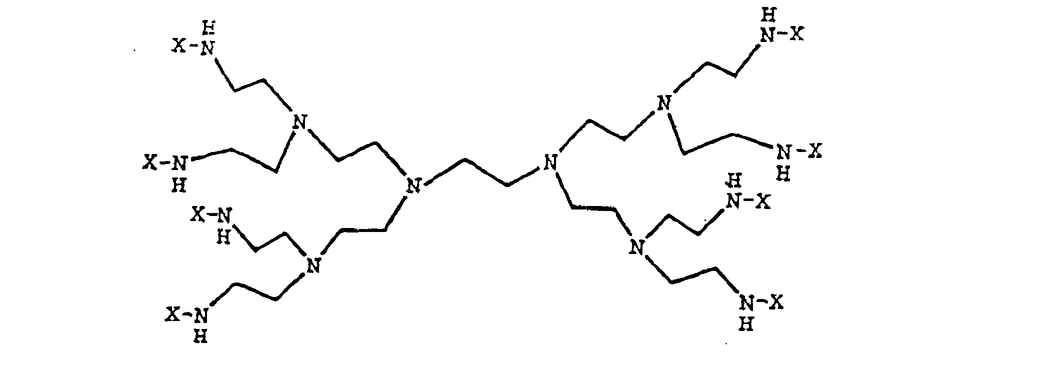 ausgewählt ist, wobei X = 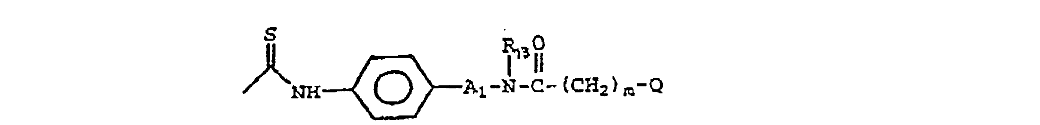 und worin Q ein Aminocarboxylat-Ligand ist und die anderen Variablen wie in Anspruch 1 definiert sind.

## Revendications (Revendications pour l'(les) Etat(s) contractant(s) suivant(s): AT, BE, CH, DE, DK, FR, GB, GR, IE, IT, LI, LU, MC, NL, PT, SE)

1. Agent de diagnostic comprenant un ligand aminocarboxylate, complexé avec un ion de métal paramagnétique, de formule Ia 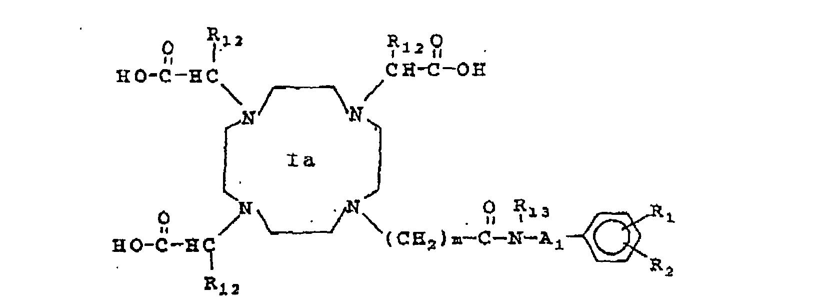 dans laquelle A₁ est -(CH₂)_{m'}- ou une liaison ;
-(CH₂)ₘ- et -(CH₂)_{m'}- peuvent indépendamment être substitués par un alkyle ou un hydroxyalkyle ;
R₁ et R₂ sont indépendamment H, un alkyle, -NO₂, -NH₂, 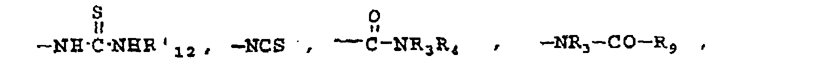 où R₉ est un alkyle ou un hydroxyalkyle, sous réserve qu'au moins un des R₁ et R₂ ne soit pas un hydrogène ;
R₃ et R₄ sont indépendamment H, un alkyle, un aralkyle, un aryle, un alcoxy, et un hydroxyalkyle ;
R₁₂ est H, un alkyle ou un hydroxyalkyle ;
R'₁₂ est H, un alkyle ou un hydroxyalkyle ; R₁₃ est H, un alkyle, un aralkyle, un aryle, un alcoxy, un hydroxyalkyle ;
m et m' valent indépendamment 1 à 5 ; et les formes multimères de celui-ci.

2. Agent de diagnostic de la revendication 1, dans lequel le métal paramagnétique est Gd.

3. Composé des formes multimères de celui de formule Ia telle que définie dans la revendication 1.

4. Composé de formule Ia, dans lequel R₁ et R₂ sont -CO-NR₃R₄, et R₃ est un hydroxyalkyle.

5. Composé de formule Ia, dans lequel R₁ et R₂ sont -CO-NHR₃, où R₃ est choisi parmi -CH₂-CHOH-CH₂OH et -CH(CH₂OH)₂.

6. Composé de formule Ia, dans lequel R₁ et R₂ sont -CONH-CH₂-CHOH-CH₂OH ou -CONH-CH(CH₂OH)₂.

7. Composés de formule Ia qui sont
l'acide 10-[2-([3,5-bis[[(2,3-dihydroxypropyl)-amino]carbonyl]phényl]amino]-2-oxoéthyl]-1,4,7,10-tétraazacyclododécane-1,4,7-triacétique ;
l'acide 10-[2-[[3,5-bis[[[2-hydroxy-1-(hydroxyméthyl)éthyl]amino]carbonyl]phénylamino]-2 -oxoéthyl]-1,4,7,10-tétraazacyclododécane-1,4,7-triacétique ;
l'acide 10-[2-[méthyl[3,5-bis[[(2-méthylbutyl)-amino]carbonyl]phényl]amino]-2-oxoéthyl]-1,4,7,10-tétraazacyclododécane-1,4,7-triacétique ;
l'acide 10-[2-[[4-[[2,3-dihydroxypropyl]amino]-carbonyl]phényl]amino]-2-oxoéthyl-1,4,7,10-tétraazacyclododécane-1,4,7-triacétique ;
l'acide 10-[N-(4-nitrophényl)acétamido]-1,4,7,10-tétraazacyclododécane-1,4,7-triacétiqpe ;
l'acide 10-[N-(4-aminophényl)acétamido]-1,4,7,10-tétraazacyclododécane-1,4,7-triacétique ;
l'acide 10-[[N-{4-(N'-isothiocyanato)phényl]-acétamido]]-1,4,7,10-tétraazacyclododécane-1,4,7-triacétique ;
l'acide 10-[N-[4-(N'-méthylthiouréido)phényl]-acétamido]-1,4,7,10-tétraazacyclododécane-1,4,7-triacétique ;
l'acide 10-[N-[4-(N',N'-diéthylaminothiouréido)-phényl]acétamido]-1,4,7,10-tétraazacyclododécane-1,4,7-triacétique ;
l'acide 10,10'-[[[[[(1,2-éthanediyl)diimino]bis-(thioxométhyl)diimino]-bis(4,1-phénylène)]diimino-bis(2-oxo-2,1-éthanediyl)]bis [1,4,7,10-tétraazacyclododécane-1,4,7-triacétique] ;
la N,N'-bis[N"-[2-(acide 1,4,7,10-tétraazacyclododécane-1,4,7-triacétique)-1-oxoéthyl]aminophényl]-thiourée ;
l'acide 10,10'-[[[[[[iminotris(1,2-éthanediyl)-triimino] tris (thioxométhyl)triimino]tris- (4,1-phénylène)]-triimino-tris(2-oxo-2,1-éthanediyl)]tris-[1,4,7,10-tétraazacyclododécane-1,4,7-triacétique] ; ou
l'acide 10-[2-[[2-(4-nitrophényl)éthyl]amino]-2-oxoéthyl]-1,4,7,10-tétraazacyclododécane-1,4,7-triacétique.

8. Complexes avec Gd des composés de la revendication 7.

9. Complexe d'un atome métallique et d'un ligand chélatant les métaux des composés selon les revendications 4-8, ou un sel pharmaceutiquement acceptable d'un tel complexe.

10. Complexe de la revendication 9, dans lequel ledit atome métallique a un numéro atomique de 56 à 83, par exemple Gd.

11. Composés multimères choisis parmi les formules 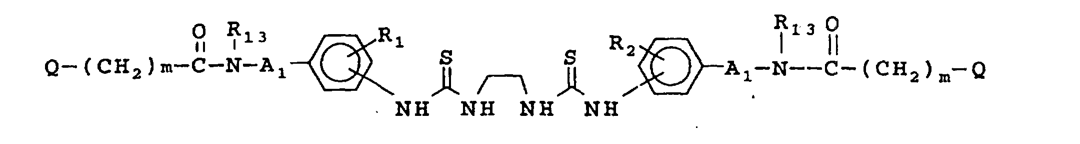 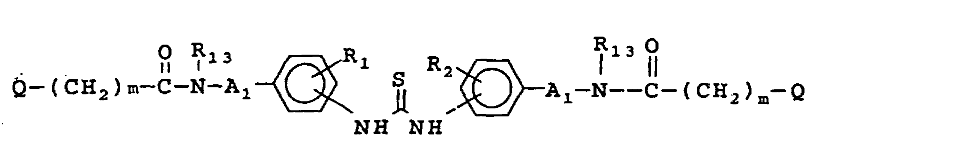 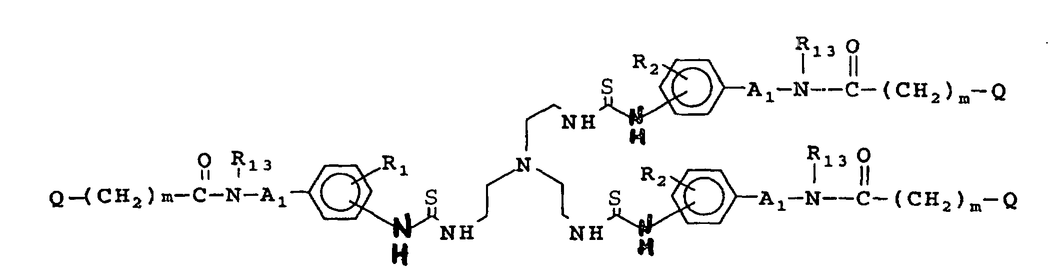 ou 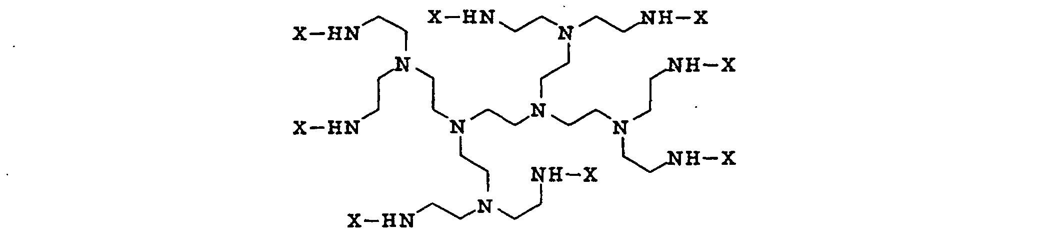 où X est un groupe de formule 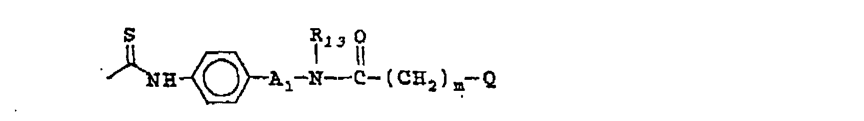 et Q est un ligand aminocarboxylate défini dans la revendication 1, les autres variables étant définies comme dans la revendication 1.

## Revendications (Revendications pour l'(les) Etat(s) contractant(s) suivant(s): ES)

1. Procédé de préparation d'un composé de formule Ia 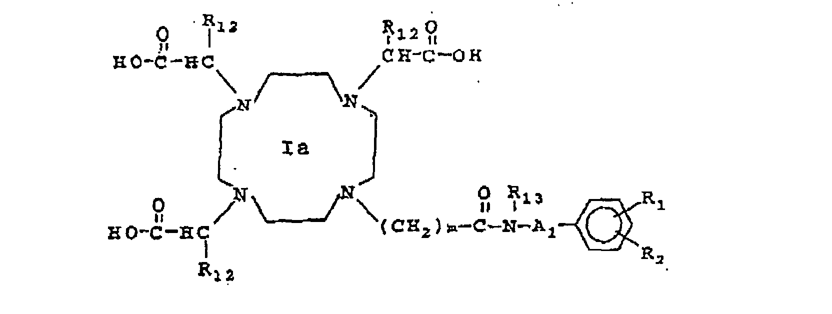 dans laquelle A₁ est -(CH₂)_{m'}- ou une liaison ;
-(CH₂)ₘ- et -(CH₂)_{m'}- peuvent indépendamment être substitués par un alkyle ou un hydroxyalkyle ;
R₁ et R₂ sont indépendamment H, un alkyle, -NO₂, -NH₂, 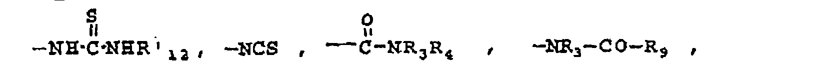 où R₉ est un alkyle ou un hydroxyalkyle, sous réserve qu'au moins un des R₁ et R₂ ne soit pas un hydrogène ;
R₃ et R₄ sont indépendamment H, un alkyle, un aralkyle, un aryle, un alcoxy, et un hydroxyalkyle ; R₁₂ est H, un alkyle ou un hydroxyalkyle ;
R'₁₂ est H, un alkyle ou un hydroxyalkyle ; R₁₃ est H, un alkyle, un aralkyle, un aryle, un alcoxy, un hydroxyalkyle ; m et m' valent indépendamment 1 à 5 ; et les formes multimères de celui-ci ;
lequel procédé comprend la réaction d'un composé de formule 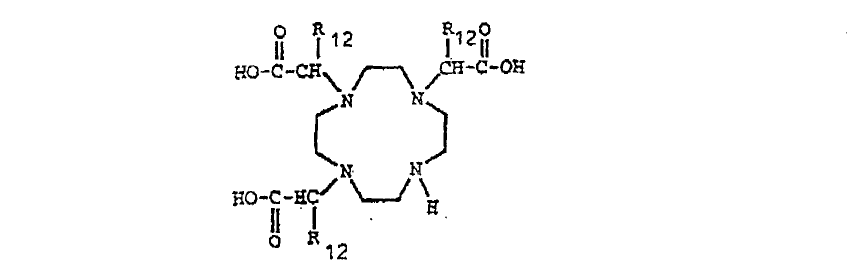 avec un composé de formule 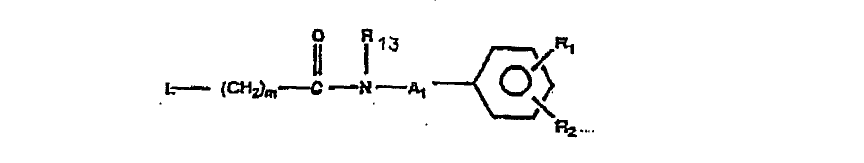 (où L est un groupe partant, par exemple un halogène) dans un solvant et en présence d'une base.

2. Procédé de la revendication 1 dans lequel R₁ et R₂ sont chacun 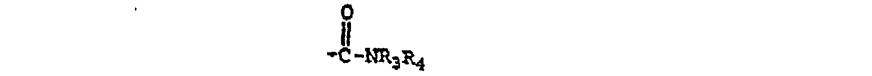 où chaque groupe R₃ est un hydroxyalkyle.

3. Procédé de la revendication 1 dans lequel R₁ et R₂ sont chacun 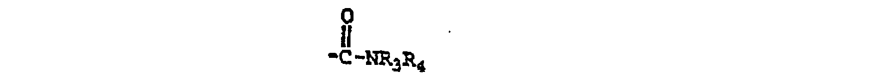 où chaque groupe R₃ est choisi parmi 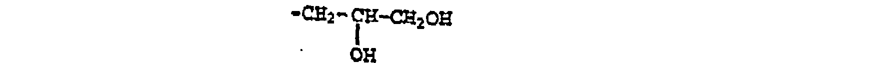 et -CH(CH₂OH)₂, et où chaque groupe R₄ est un hydrogène.

4. Procédé de la revendication 1 dans lequel R₁ et R₂ sont chacun

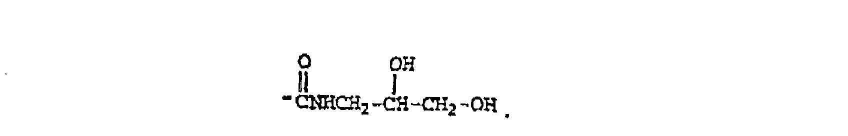

5. Procédé de la revendication 1 dans lequel R₁ et R₂ sont chacun

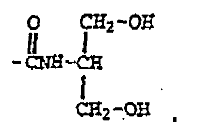

6. Procédé de la revendication 1 dans lequel le produit est l'acide 10-[2-[[3,5-bis [[(2,3-dihydroxypropyl)amino]carbonyl]phényl]amino]-2-oxoéthyl]-1,4,7,10-tétraazacyclododécane-1,4,7-triacétique ;
l'acide 10-[2-[[3,5-bis[[[2-hydroxy-1-(hydroxyméthyl)éthyl]amino]carbonyl]phénylamino]-2-oxoéthyl]-1,4,7,10-tétraazacyclododécane-1,4,7-triacétique ;
l'acide 10-[2-[méthyl[3,5-bis[[(2-méthylbutyl)-amino]carbonyl]phényl]amino]-2-oxoéthyl]-1,4,7,10-tétraazacyclododécane-1,4,7-triacétique ;
l'acide 10-[2-[[4-[[2,3-dihydroxypropyl]amino]-carbonyl]phényl]amino]-2-oxoéthyl-1,4,7,10-tétraazacyclododécane-1,4,7-triacétique ;
l'acide 10-[N-(4-nitrophényl)acétamido]-1,4,7,10-tétraazacyclododécane-1,4,7-triacétique ;
l'acide 10-[N-(4-aminophényl)acétamido]-1,4,7,10-tétraazacyclododécane-1,4,7-triacétique ;
l'acide 10-[[N-{4-(N'-isothiocyanato)phényl]-acétamido]]-1,4,7,10-tétraazacyclododécane-1,4,7-triacétique ;
l'acide 10-[N-[4-(N'-méthylthiouréido)phényl]-acétamido]-1,4,7,10-tétraazacyclododécane-1,4,7-triacétique ;
l'acide 10-[N-[4-(N',N'-diéthylaminothiouréido)-phényl]acétamido]-1,4,7,10-tétraazacyclododécane-1,4,7-triacétique ;
l'acide 10,10'-[[[[[(1,2-éthanediyl)diimino]bis-(thioxométhyl)diimino]-bis(4,1-phénylène)]diimino-bis(2-oxo-2,1-éthanediyl)]bis[1,4,7,10-tétraazacyclododécane-1,4,7-triacétique] ;
la N,N'-bis[N"-[2-(acide 1,4,7,10-tétraazacyclododécane-1,4,7-triacétique)-1-oxoéthyl]aminophényl]-thiourée ;
l'acide 10,10'-[[[[[[iminotris(1,2-éthanediyl)-triimino] tris(thioxométhyl)triimino]tris-(4,1-phénylène)]-triimino-tris(2-oxo-2,1-éthanediyl)]tris-[1,4,7,10-tétraazacyclododécane-1,4,7-triacétique] ; ou
l'acide 10-[2-[[2-(4-nitrophényl)éthyl]amino]-2-oxoéthyl]-1,4,7,10-tétraazacyclododécane-1,4,7-triacétique.

7. Procédé de la revendication 1 ou 2 dans lequel l'ion de métal paramagnétique est le gadolinium et le ligand est un composé nommé dans la revendication 6.

8. Procédé de préparation d'un complexe d'un ligand aminocarboxylate et d'un ion métallique, ou d'un sel pharmaceutiquement acceptable d'un tel complexe, dans lequel le ligand aminocarboxylate est un composé de formule la telle que définie dans l'une quelconque des revendications 1 à 5, lequel procédé comprend la réaction du ligand aminocarboxylate avec une source de l'ion métallique.

9. Procédé de la revendication 8 dans lequel ledit atome métallique a un numéro atomique de 56 à 83.

10. Procédé de la revendication 8 dans lequel ledit métal est le gadolinium (III).

11. Procédé de la revendication 1 dans lequel le produit est un multimère choisi parmi 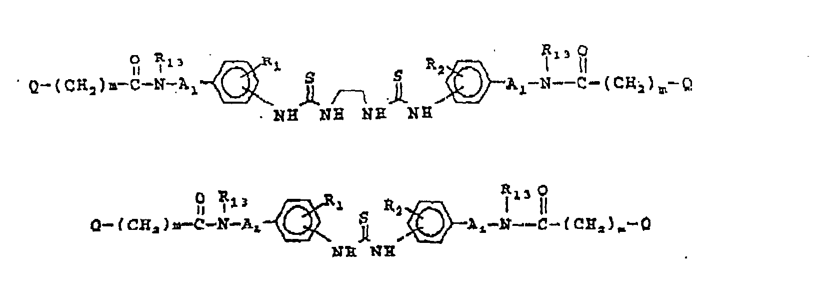 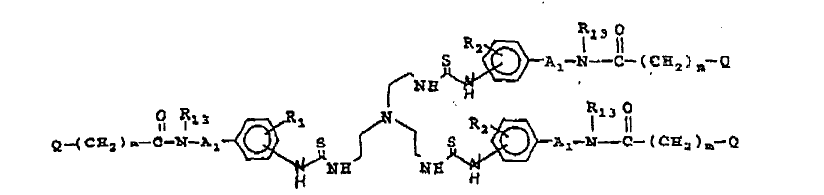 ou 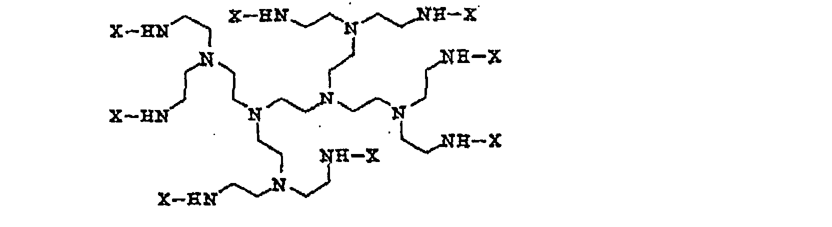 où X = 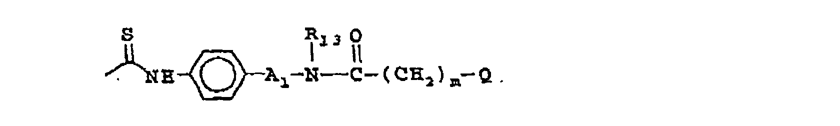 et où Q est un ligand aminocarboxylate et les autres variables sont telles que définies dans la revendication 1.
